# EUROPEAN PATENT APPLICATION

(11) **EP 4 338 741 A1**
(43) Date of publication of application: **20.03.2024**
(21) Application number: 22806728.6
(22) Date of filing: 10.05.2022
(51) Int. Cl.: A61K 31/551, C07D 471/14, A61P 35/00

(54) **PHARMACEUTICAL COMPOSITION AND USE OF MULTI-KINASE INHIBITOR**

(30) Priority: 10.05.2021 CN 202110514716
(71) Applicant: Transthera Sciences (Nanjing), Inc., Jiangbei New Area Nanjing Jiangsu 210032 (CN)
(72) Inventor: PENG, Peng, Nanjing, Jiangsu 210032 (CN); QIANG, Xiaoyan, Nanjing, Jiangsu 210032 (CN); SUN, Caixia, Nanjing, Jiangsu 210032 (CN); WANG, Hui, Nanjing, Jiangsu 210032 (CN); YU, Qi, Nanjing, Jiangsu 210032 (CN)
(74) Representative: Grünecker Patent- und Rechtsanwälte PartG mbB
(86) International application number: PCT/CN2022/091924
(87) International publication number: WO 2022/237765

(57) **Abstract**

A pharmaceutical composition and use of a multi-kinase inhibitor. The present invention relates to the technical field of medicines, and relates to the pharmaceutical composition and use of the multi-kinase inhibitor. The pharmaceutical composition comprises: (a) a compound represented by general formula (I) or a pharmaceutically acceptable salt, a stereoisomer or a crystal form thereof, 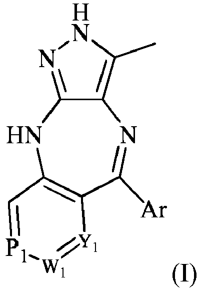 wherein P₁, W₁, Y₁, and Ar in general formula (I) are as described herein; and (b) a paclitaxel drug. Variables in the general formula are as defined in the description. Studies have shown that the compound of general formula (I) or the pharmaceutically acceptable salt, stereoisomer or crystal form thereof in the present invention has a significant synergistic interaction with the paclitaxel drug in combination for treating cancers.

## Description

### TECHNICAL FIELD

The present invention belongs to the technical field of pharmaceuticals, and particularly relates to a pharmaceutical composition of a multi-kinase inhibitor and use thereof.

### BACKGROUND

Cancer is a disease caused by the abnormal regulation and hyperproliferation of body cells. The most common childhood cancers are leukemia, lymphoma, brain tumor, and bone cancer, while adult cancers are mostly lung cancer, colon cancer, breast cancer, prostate cancer, and pancreatic cancer. Nowadays, with the increasing incidence rate of cancer, it is a serious threat to human health.

Triple negative breast cancer (TNBC) is a subtype of breast cancer that appears to be negative for estrogen receptor (ER), progestogen receptor (PR), and human epidermal receptor 2 (HER2), accounting for approximately 15%-20% of the total breast cancer patients. Due to the lack of the above three receptors, patients with TNBC received hormone anti-tumor therapies (such as tamoxifen or aromatase inhibitors), HER2 receptor-targeted therapies (trastuzumab, T-DM1, pertuzumab), or CDK4/6 inhibitors (such as palbociclib, ribociclib, and abemaciclib), but none of them showed alleviation. In recent years, three therapies have been approved for systemic treatment of TNBC, including atezolizumab combined with albumin paclitaxel for TNBC patients with high expression of PD-L1 (approved in 2019), Trodelvy (sacituzumab govitecan-hziy) for metastatic triple negative breast cancer (mTNBC) patients who have previously received at least two therapies for treating metastatic diseases (approved in 2020), and pembrolizumab (Keytruda) combined with chemotherapy for treating TNBC patients with high expression of PD-L1 (approved in 2020). However, due to the high heterogeneity of TNBC, there is still a lack of effective therapeutic drugs, and there is an urgent need to develop more effective treatment strategies.

Lung cancer is the cancer with the highest incidence rate and mortality in the world, and is mainly divided into non-small cell lung cancer (NSCLC) and small cell lung cancer (SCLC). Non-small cell lung cancer accounts for approximately 85% of lung cancers, which can be further divided into adenocarcinoma, squamous cell carcinoma, and large cell carcinoma. At the time of definite diagnosis, the diseases of most patients have progressed to the moderate or advanced stage, making treatment even more difficult. At present, the main clinical treatment methods for lung cancer include chemotherapy, intervention, surgery, gene therapy, immunotherapy, targeted therapy, etc. The chemotherapeutics commonly used in clinic at present include cisplatin, gemcitabine, doxorubicin, paclitaxel, vincristine, etc. Although there are many drugs used to treat lung cancer, the survival rate of patients has not been obviously improved, and chemotherapy is palliative, with the main purpose of prolonging the survival of the patients and improving the life quality of the patients. In recent years, with the progress and development of the field of pharmacology, there have been more and more intervention measures for treating cancer, including surgery, chemotherapy, hormone therapy, radiotherapy, immunotherapy, gene therapy, etc., wherein the surgery, radiotherapy, and chemotherapy are the most commonly used treatment means.

For the common chemotherapy means, paclitaxel drug is the most widely used and important chemotherapeutic drug, which has a unique anti-tumor mechanism and mainly acts on tubulin. Tubulin is a component constituting the cytoskeleton of all eukaryotic cells and is also an important component constituting the spindle during cell division. Paclitaxel increases the content of tubulin in cells by inducing and stabilizing tubulin polymerization and inhibiting its normal physiological depolymerization. In the process of cell division, the increase of tubulin will lead to the reduction of spindle microtubules, and spindle fibers and spindle apparatus cannot be formed, so that division of cancer cells is always kept in G2 and M stages, leading to their inability to replicate, and ultimately leading to apoptosis. At present, there are mainly four kinds of paclitaxel drugs in clinic: paclitaxel injection, docetaxel, paclitaxel liposome for inj ection, and paclitaxel for inj ection (albumin bound), which are used alone or in combination with other anti-tumor drugs, mainly for the treatment of various cancers such as breast cancer, ovarian cancer, non-small cell lung cancer, and pancreatic cancer. Nevertheless, the adverse effects caused by a paclitaxel drug affect different systems and organs, such as the blood system, nervous system, cardiovascular system, digestive system, skin, and accessories thereof. The most common adverse effects are myelosuppression, anaphylaxis, and neurotoxicity. Dose factor is the most important factor that causes adverse effects of a paclitaxel drug. The incidence rate and severity of the adverse reactions are particularly obvious at high doses, and there is no effective means for alleviating such adverse effects at present. In addition, the emergence of resistance to paclitaxel also limits its clinical application.

Based on the strong side effects of chemotherapy and the lack of more effective treatment means for various cancers such as breast cancer, cholangiocarcinoma, and lung cancer, the development of more effective treatment means has become a problem to be urgently solved.

### SUMMARY

A compound of general formula (I) in the present invention is a multi-kinase inhibitor targeting cell proliferation, angiogenesis, and tumor immune signaling pathways, and these pathways play a key role in regulating cytokine signaling and angiogenesis, possibly preventing the growth of tumor cells by blocking proteins required for cell growth, and possibly preventing the neovascularization required for tumor growth. It is found by study that the combination of the multi-kinase inhibitor of the present invention and a paclitaxel drug has a significant synergistic treatment effect on cancer.

In order to achieve the above objective, the present invention first provides the following solutions:

The present invention provides a pharmaceutical composition, comprising: (a) a compound of general formula (I) or a pharmaceutically acceptable salt, a stereoisomer or a crystal form thereof, and (b) a paclitaxel drug, 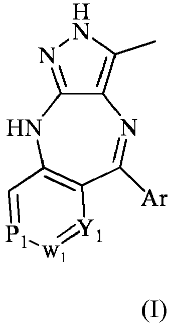
wherein Ar is phenyl optionally substituted with 1 to 3 R⁶, and each R⁶ is independently selected from hydrogen, amino, cyano, halogen, C₁₋₄ alkyl, and trifluoromethyl;
Y₁ is CR³;
P₁ is CR⁴;
W₁ is N;
R³ is hydrogen or C₁₋₄ alkyl;
R⁴ is -(CH₂)ₙ-(5-11) membered heterocyclyl, wherein n = an integer of 0 to 6, a ring-forming S atom (if any) in the heterocyclyl is optionally oxidized to S(O) or S(O)₂, a ring-forming C atom in the heterocyclyl is optionally oxidized to C(O), and the heterocyclyl is optionally substituted with one or more substituents independently selected from C₁₋₃ alkyl and C₃₋₆ cycloalkyl.

In one embodiment, Ar is phenyl optionally substituted with 1 to 3 R⁶, and each R⁶ is independently selected from hydrogen and halogen;
R³ is hydrogen;
R⁴ is selected from -(CH₂)ₙ-(5-6) membered monocyclic heterocyclyl and - (CH₂)ₙ-(7-11) membered fused heterocyclyl, wherein n = an integer of 0 to 6, a ring-forming S atom (if any) in the heterocyclyl is optionally oxidized to S(O) or S(O)₂, a ring-forming C atom in the heterocyclyl is optionally oxidized to C(O), and the heterocyclyl is optionally substituted with one or more substituents independently selected from C₁₋₃ alkyl and C₃₋₆ cycloalkyl.

In a further embodiment, the (5-6) membered monocyclic heterocyclyl is (5-6) membered saturated monocyclic heterocyclyl, and the (7-11) membered fused heterocyclyl is (7-11) membered saturated fused heterocyclyl. In a preferred embodiment, the (7-11) membered saturated fused heterocyclyl is (7-11) membered saturated ortho-fused heterocyclyl, (7-11) membered saturated spiro-heterocyclyl, or (7-11) membered saturated bridged heterocyclyl.

In one embodiment, R⁴ is 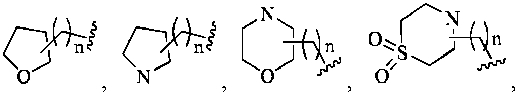 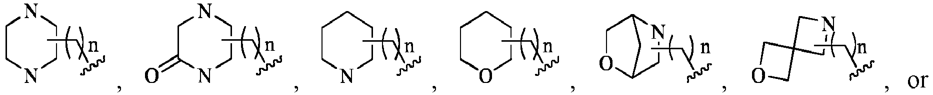 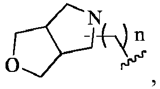 and n = an integer of 0 to 3, wherein the heterocyclyl in R⁴ is optionally substituted with one or more substituents independently selected from C₁₋₃ alkyl and C₃₋₆ cycloalkyl (for example, 1, 2, or 3 substituents independently selected from C₁₋₂ alkyl and C₃ cycloalkyl); for example, R⁴ is 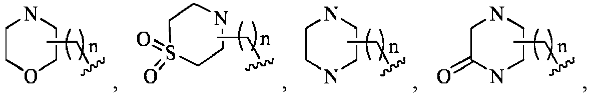 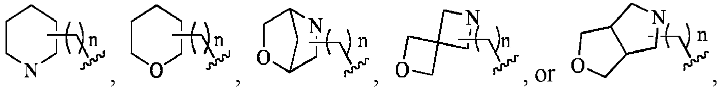 and n = an integer of 0 to 3, wherein the heterocyclyl in R⁴ is optionally substituted with one or more substituents independently selected from C₁₋₃ alkyl and C₃₋₆ cycloalkyl (for example, 1, 2, or 3 substituents independently selected from C₁₋₂ alkyl and C₃ cycloalkyl).

In a preferred embodiment, R⁴ is preferably 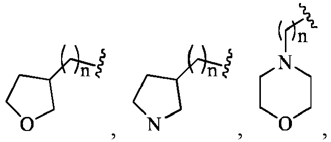 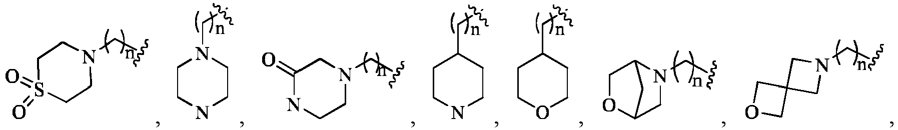 or 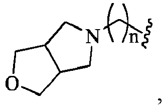 and n = an integer of 0 to 3, wherein the heterocyclyl in R⁴ is optionally substituted with one or more substituents independently selected from C₁₋₃ alkyl and C₃₋₆ cycloalkyl (for example, 1, 2, or 3 substituents independently selected from C₁₋₂ alkyl and C₃ cycloalkyl); for example, R⁴ is preferably 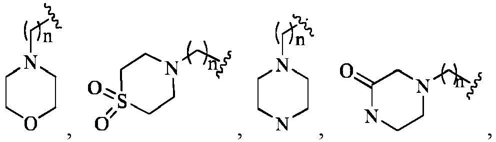 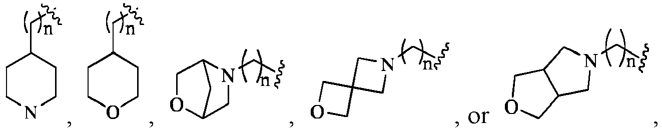 and n = an integer of 0 to 3, wherein the heterocyclyl in R⁴ is optionally substituted with one or more substituents independently selected from C₁₋₃ alkyl and C₃₋₆ cycloalkyl (for example, 1, 2, or 3 substituents independently selected from C₁₋₂ alkyl and C₃ cycloalkyl).

In one embodiment, the compound of general formula (I) includes the compounds shown in Table 1.

**Table 1: Examples of the compound of general formula (I)**

| Compound | Structure | Compound | Structure |
|---|---|---|---|
| 22 | 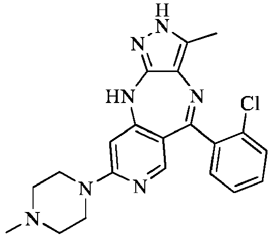 | 29 | 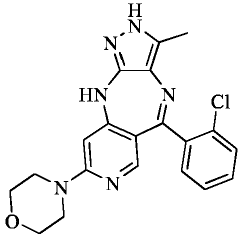 |
| 33 | 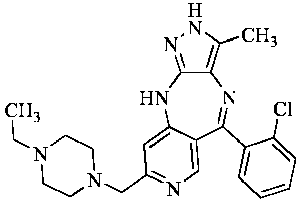 | 34 | 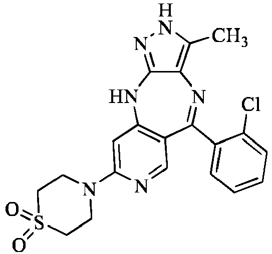 |
| 35 | 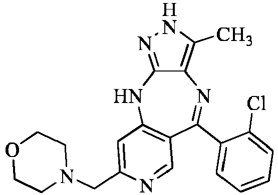 | 36 | 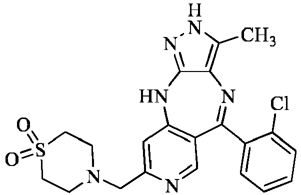 |
| 37 | 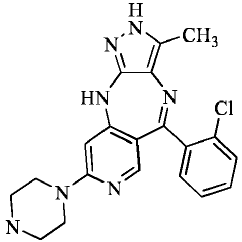 | 38 | 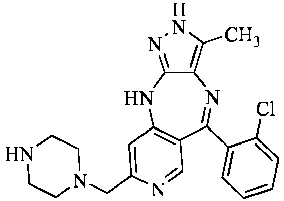 |
| 39 | 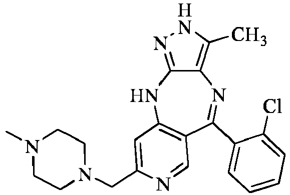 | 40 | 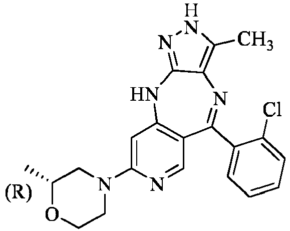 |
| 41 | 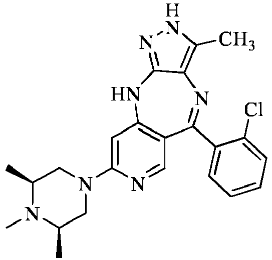 | 42 | 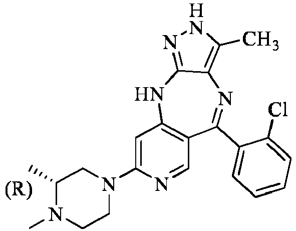 |
| 43 | 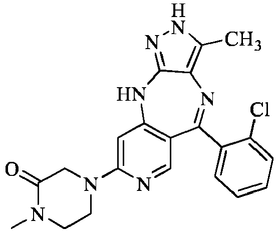 | 44 | 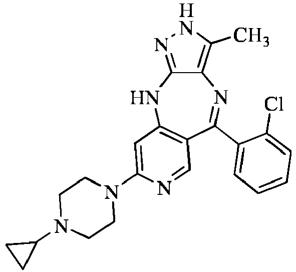 |
| 45 | 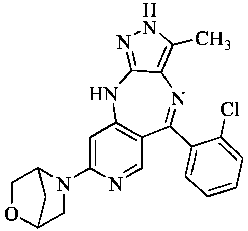 | 46 | 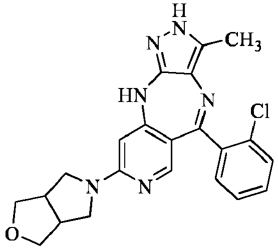 |
| 47 | 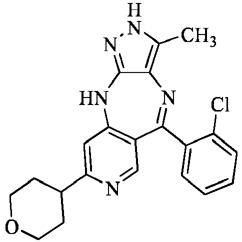 | 48 | 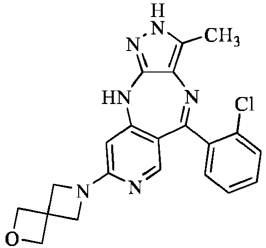 |
| 49 | 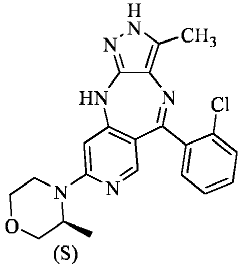 | 50 | 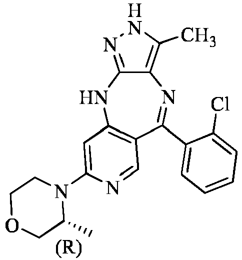 |
| 51 | 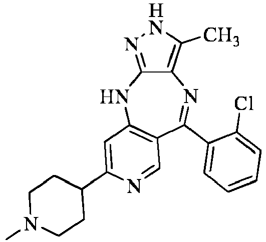 | | |

In one embodiment, the compound of the present invention may also include compounds other than the compound of general formula (I), for example, the specific compounds shown in the table below.

| Compound | Structure | Compound | Structure |
|---|---|---|---|
| 1 | 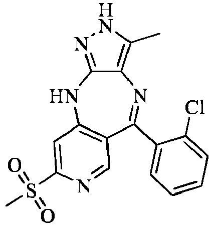 | 2 | 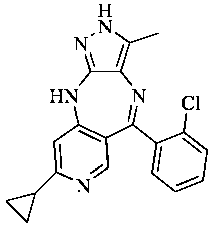 |
| 3 | 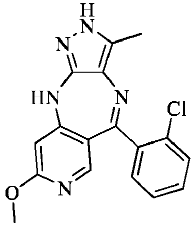 | 4 | 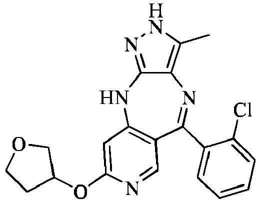 |
| 5 | 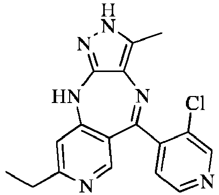 | 6 | 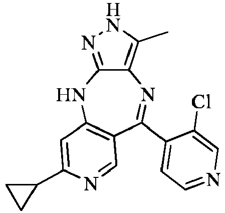 |
| 7 | 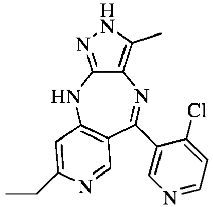 | 8 | 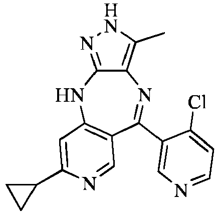 |
| 9 | 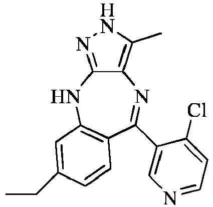 | 10 | 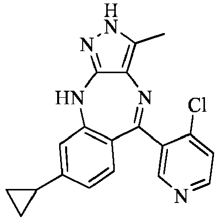 |
| 11 | 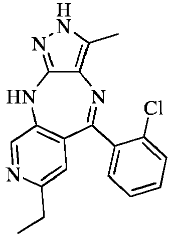 | 12 | 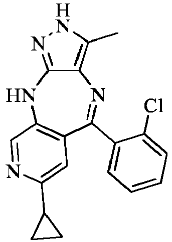 |
| 13 | 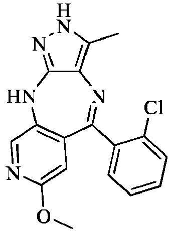 | 14 | 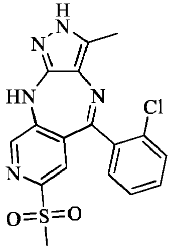 |
| 15 | 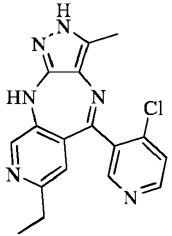 | 16 | 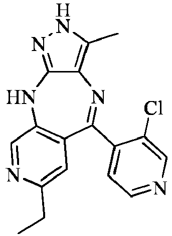 |
| 17 | 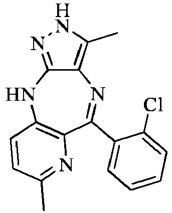 | 18 | 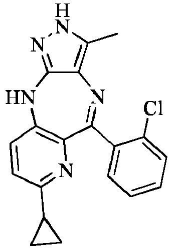 |
| 19 | 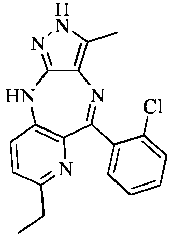 | 20 | 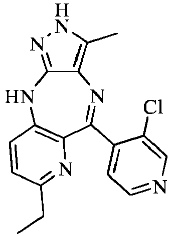 |
| 21 | 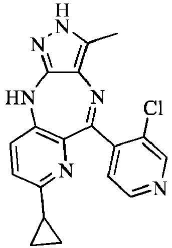 | 30 | 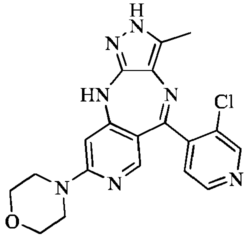 |
| 23 | 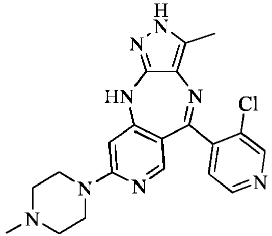 | 24 | 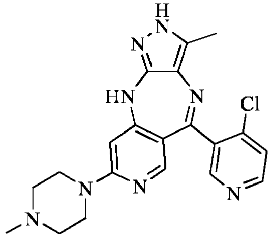 |
| 25 | 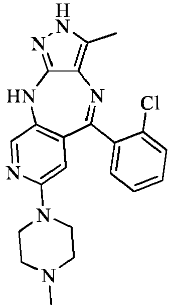 | 26 | 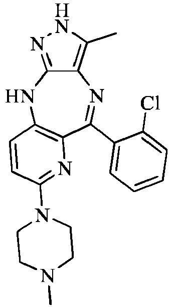 |
| 27 | 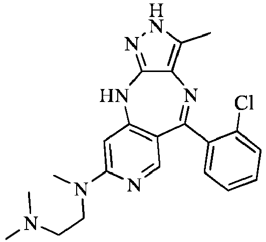 | 28 | 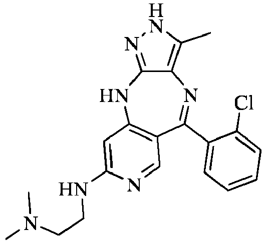 |
| 31 | 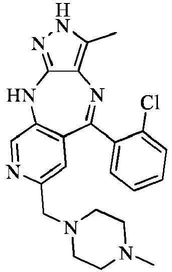 | 32 | 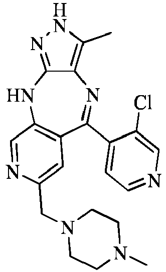 |

In one embodiment, the paclitaxel drug is selected from one or more of paclitaxel, paclitaxel for injection (albumin bound), paclitaxel liposome for injection, and docetaxel.

In one embodiment, the compound is 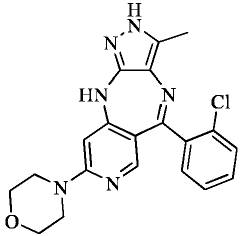 or a pharmaceutically acceptable salt, a stereoisomer or a crystal form thereof; the paclitaxel drug is paclitaxel for injection (albumin bound) or paclitaxel.

In one embodiment, the paclitaxel drug is selected from paclitaxel and analogs and formulations thereof.

In one embodiment, the paclitaxel drug is selected from one or more of paclitaxel, docetaxel, and cabazitaxel.

In the present invention, the "paclitaxel drug" refer to molecules belonging to the taxane family of anti-tumor drugs. Molecules of the taxane family bind to β-tubulin and stabilize the polymerization form of microtubules. Therefore, they act as mitosis inhibitors by destroying microtubule function.

The mechanism of action of paclitaxel is to stabilize microtubules by promoting the combination of tubulin dimers and preventing their depolymerization, thereby inhibiting the normal dynamic reorganization of microtubules which is crucial for cell functions at the interphase and mitotic phase, hindering the replication of tumor cells, and making cancer cells unable to continue to divide and thus die. In addition, paclitaxel also has a radiosensitization effect, which can promote cell damage caused by ion irradiation.

In the present invention, paclitaxel analog refers to active ingredients with a taxane skeleton structure or modified derivatives thereof, for example, docetaxel and cabazitaxel, which are similar to paclitaxel in the anti-tumor mechanism, but have their own characteristics and advantages.

In one embodiment, the paclitaxel drug is selected from:
paclitaxel such as Taxol^{®}, Aosu^{®}, Anzatax^{®}, Tesu^{®}, Funeng^{®}, Zisu^{®}, Zexin^{®}, Anxiao^{®}, Furuitai^{®}, and Paclitaxel Kabi^{®};
paclitaxel liposome for injection such as Lipusu^{®};
paclitaxel for injection (albumin bound) (nab-paclitaxel), such as Keruifei^{®} and Qiluruibei^{®};
nab-paclitaxel, such as Abraxane^{®}, Aiyue^{®}, and Keaili^{®};
docetaxel, such as Taxotere^{®}, Aomingrun^{®}, Aisu^{®}, Siqudi^{®}, Duopafei^{®}, Chenhuan^{®}, Yiyouruikang^{®}, and Xicun^{®};

In one embodiment, the paclitaxel drug is cabazitaxel (such as Jevtana^{®}), polyglutamic acid paclitaxel (Opaxio^{®}), BMS-275183, ortataxel, or BMS-184476.

In one embodiment, the paclitaxel analog includes docetaxel, cabazitaxel (such as Jevtana^{®}), larotaxel, polyglutamic acid paclitaxel (Opaxio^{®}), BMS-275183, ortataxel, BMS-184476, paclitaxel trevatide, Salutaxel, Conmutaxol, felotaxel, BMS-188797, milataxel, tesetaxel, and docosahexaenoic acid-paclitaxel (DHA-paclitaxel).

In one embodiment, the paclitaxel drug is classified by formulation, and the paclitaxel includes, for example, paclitaxel injection, paclitaxel liposome for injection, paclitaxel for injection (albumin bound), paclitaxel oral liquid, paclitaxel capsule, paclitaxel cationic liposome EndoTAG-1, paclitaxel micelle, and paclitaxel polymer micelle. The formulation of the docetaxel includes docetaxel injection, docetaxel liposome, docetaxel microsphere injection, docetaxel polymer micelle, etc. The formulations of the above other paclitaxel analogs are similar to those of paclitaxel and docetaxel.

The present invention also provides a combination formulation, comprising the compound or the pharmaceutically acceptable salt, the stereoisomer or the crystal form thereof of the present invention, and the paclitaxel drug, and a pharmaceutically acceptable carrier.

The present invention also provides a kit, comprising the pharmaceutical composition or combination formulation described above, and a package insert.

Further, the kit described herein comprises a) one or more unit formulations of the compound or the pharmaceutically acceptable salt, the stereoisomer or the crystal form thereof of the present invention; b) one or more unit formulations of a paclitaxel drug; and c) a package insert.

The present invention also provides use of the pharmaceutical composition, the combination formulation, or the kit described above in the manufacture of a medicament for preventing and/or treating a cancer.

In one embodiment, the use further includes that the compound of general formula (I) or the pharmaceutically acceptable salt, the stereoisomer or the crystal form thereof, and the paclitaxel drug in the pharmaceutical composition, the combination formulation, or the kit are administered in combination to a cancer patient in therapeutically effective amounts simultaneously, separately, or sequentially.

In one embodiment, the present invention also provides a method for combined treatment of a cancer, comprising administering to a cancer patient in need an effective amount of the pharmaceutical composition, the combination formulation, or the kit comprising the compound of general formula (I) or the pharmaceutically acceptable salt, the stereoisomer or the crystal form thereof, and the paclitaxel drug.

In one embodiment, the present invention also provides use of the pharmaceutical composition, the combination formulation, or the kit comprising the compound of general formula (I) or the pharmaceutically acceptable salt, the stereoisomer or the crystal form thereof, and the paclitaxel drug in preventing and/or treating a cancer.

The "treat, treating, or treatment" described herein refers to administering to a patient an effective amount of a medicament that slows or cures an undesired physiological change or condition in the patient, such as a hyperproliferative condition, such as the growth, formation, or spread of a cancer, to achieve a favorable or desired clinical result, including, but not limited to: alleviation of symptoms, diminishment of the disease, stabilization (i.e., not worsening) of the disease state, delay or slowing of the disease progression, amelioration or palliation of the disease state, and regression (whether partial or complete) of the disease, whether detectable or undetectable.

"Treat, treating, or treatment" described herein may include neoadjuvant treatment and adjuvant treatment.

The "neoadjuvant treatment" refers to systemic treatment for a patient with an original tumor prior to planned surgery or local treatment with surgery plus radiotherapy, and treatment means of the neoadjuvant treatment may be chemotherapy, endocrine, targeting, immunization, and radiotherapy depending on the tumor types. The purpose of neoadjuvant treatment is to provide immediate systemic treatment, thereby potentially eradicating micrometastases that otherwise proliferate when following the standard sequence of surgery followed by systemic treatment. The neoadjuvant treatment may also help reduce tumor size, allowing complete resection of an initially unresectable tumor or preservation of portions of the organ and its functions. In addition, the neoadjuvant treatment allows for *in vivo* assessment of the efficacy of a medicament, which may guide the choice of subsequent treatments.

The "adjuvant treatment" refers to a treatment given after definitive surgery (where evidences of residual diseases cannot be detected) to destroy any residual cancer cells in a body, for use in the reduction of the likelihood of tumor relapse or dissemination to other sites. Treatment means of the adjuvant treatment are about the same as those of the neoadjuvant treatment. The objective of the adjuvant treatment is to prevent cancer relapse and thus reduce the chance of cancer-related death. The adjuvant treatment specifically excludes the neoadjuvant treatment herein.

The "combination", "combination formulation", "used in combination", "combination therapy", or "combined treatment" described herein means that at least one dose of the compound of general formula (I) or the pharmaceutically acceptable salt, the stereoisomer or the crystal form thereof described above and the paclitaxel drug described above is administered over a certain period of time, which can be co-prepared into a compound formulation, or each separately prepared into a formulation for simultaneous, separate, or sequential administration to a patient.

The compound or the pharmaceutically acceptable salt, the stereoisomer or the crystal form thereof of the present invention and the paclitaxel drug in the combination formulation of the present invention can each be separately prepared into a formulation, or co-prepared into a compound formulation, and any formulation can comprise one or more pharmaceutically acceptable carriers.

The "therapeutically effective amount" described herein refers to an amount of the compound or the pharmaceutically acceptable salt, the stereoisomer and the crystal form thereof of the present invention and the above paclitaxel drug that, when administered to a patient, is at least capable of alleviating symptoms of the patient's condition. An actual amount comprising the "therapeutically effective amount" will vary depending on a variety of circumstances, including, but not limited to, the particular condition being treated, the severity of the condition, the physique and health status of the patient, and the route of administration. For example, a single bolus can be administered, several separate doses can be administered overtime, or the dose can be proportionally reduced or increased as indicated by the urgent need for treatment. It should be noted that the dose values may vary depending on the type and severity of the condition to be alleviated, and can include single or multiple doses. For any particular individual, the specific administration regimen will be adjusted over time according to the individual's needs and the professional judgment of a person administering a composition or supervising the administration of a composition.

"Unit formulation" described herein refers to a physically discrete unit suitable for use as a unit dose for human subjects and other mammals, each unit containing a predetermined amount of active substance calculated to produce the desired therapeutic effect, as well as a suitable pharmaceutical excipient. Typical unit formulations include pre-filled, pre-measured ampoules or syringes for liquid compositions, or, in the case of solid compositions, pills, tablets, capsules, lozenges, etc. In such a composition, the active substance is usually a component with a small amount (about 0.1% to about 50% by weight or preferably about 1% to about 40% by weight), with the balance being various vehicles or carriers and processing aids that contribute to the formation of the desired dosage form. The unit dose formulation is preferably about 4, 5, 6, 7, 8, 9, 10, 25, 50, 100, 250, 500, or 1000 mg per unit. In a specific embodiment, the unit formulation is packaged in a multipack for subsequent use, for example, a blisterpack containing at least 6, 9, or 12 unit formulation tablets.

The "pharmaceutically acceptable carrier" described herein includes, but is not limited to, solid carriers and liquid carriers. Suitable solid carriers include, but are not limited to, cellulose, glucose, lactose, mannitol, magnesium stearate, magnesium carbonate, sodium carbonate, sodium saccharin, sucrose, dextrin, talc, starch, pectin, gelatin, tragacanth, arabic gum, sodium alginate, *p*-hydroxylbenzoate, methylcellulose, sodium carboxymethyl cellulose, low-melting wax, cocoa butter, etc. Suitable liquid carriers include, but are not limited to, water, ethanol, polyol (such as glycerin, propylene glycol, and liquid polyethylene glycol), vegetable oil, glyceride, and mixtures thereof.

In one embodiment, the cancer is a solid tumor.

In one embodiment, the cancer is ovarian cancer, endometrial cancer, cervical cancer, head and neck cancer, nasopharyngeal cancer, esophageal cancer, lung cancer, breast cancer, gastric cancer, biliary tract tumor, pancreatic cancer, bladder cancer, or melanoma.

In the present invention, the endometrial cancer includes advanced recurrent endometrial cancer; the head and neck cancer includes first-line recurrent and metastatic head and neck squamous carcinoma; the nasopharyngeal cancer includes recurrent and metastatic nasopharyngeal cancer; the esophageal cancer includes esophageal squamous carcinoma and metastatic esophageal cancer; the lung cancer includes small cell lung cancer, unresectable non-small cell lung cancer, non-squamous non-small cell lung cancer, and squamous non-small cell lung cancer; the breast cancer includes HER2-positive/negative breast cancer and HER2-2 positive/negative advanced breast cancer; the gastric cancer includes advanced metastatic gastric cancer; the biliary tract tumor includes malignant biliary tract tumor and advanced biliary tract malignant tumor (according to the anatomical location, biliary tract tumors can be divided into cholangiocarcinoma and gallbladder carcinoma); the pancreatic cancer includes borderline resectable pancreatic cancer and metastatic pancreatic cancer; and the bladder cancer includes metastatic bladder urothelial carcinoma.

In one embodiment, the breast cancer is one after the failure of standard treatment, and/or is metastatic, and/or recurrent, or locally advanced, or advanced breast cancer.

In one embodiment, the breast cancer is HER2-negative breast cancer after the failure of standard treatment.

In one embodiment, the breast cancer is metastatic HER2-negative breast cancer.

In one embodiment, the breast cancer is locally advanced or advanced HER2-negative breast cancer.

In one embodiment, the breast cancer is recurrent HER2-negative breast cancer. In one embodiment, the breast cancer is HER2-negative breast cancer. In one embodiment, the breast cancer is triple negative breast cancer.

In one embodiment, the breast cancer is triple negative breast cancer after the failure of standard treatment.

In one embodiment, the breast cancer is metastatic triple negative breast cancer.

In one embodiment, the breast cancer is locally advanced or advanced triple negative breast cancer.

In one embodiment, the breast cancer is recurrent triple negative breast cancer.

In one embodiment, the paclitaxel drug is administered by injection, for example, intravenously, for more than 30 min. The compound of general formula (I) or the pharmaceutically acceptable salt, the stereoisomer or the crystal form thereof is administered orally, further orally on an empty stomach.

In one embodiment, the compound of general formula (I) or the pharmaceutically acceptable salt, the stereoisomer or the crystal form thereof is administered at a single dose of 3 mg to 20 mg, for example, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, or 20 mg, and is administered once daily or once every two days, preferably once daily.

In one embodiment, the paclitaxel drug is administered at a single dose of 50 mg/m² to 400 mg/m², for example, 50 mg/m², 55 mg/m², 60 mg/m², 65 mg/m², 70 mg/m², 75 mg/m², 80 mg/m², 85 mg/m², 90 mg/m², 95 mg/m², 100 mg/m², 105 mg/m², 110 mg/m², 115 mg/m², 120 mg/m², 125 mg/m², 130 mg/m², 135 mg/m², 140 mg/m², 145 mg/m², 150 mg/m², 155 mg/m², 160 mg/m², 165 mg/m², 170 mg/m², 175 mg/m², 180 mg/m², 185 mg/m², 190 mg/m², 195 mg/m², 200 mg/m², 205 mg/m², 210 mg/m², 215 mg/m², 220 mg/m², 225 mg/m², 230 mg/m², 235 mg/m², 240 mg/m², 245 mg/m², 250 mg/m², 255 mg/m², 260 mg/m², 265 mg/m², 270 mg/m², 275 mg/m², 280 mg/m², 285 mg/m², 290 mg/m², 295 mg/m², 300 mg/m², 305 mg/m², 310 mg/m², 315 mg/m², 320 mg/m², 325 mg/m², 330 mg/m², 335 mg/m², 340 mg/m², 345 mg/m², 350 mg/m², 355 mg/m², 360 mg/m², 365 mg/m², 370 mg/m², 375 mg/m², 380 mg/m², 385 mg/m², 390 mg/m², 395 mg/m², or 400 mg/m², or any value between 50 mg/m² and 400 mg/m², with the recommended single dose being 100 mg/m² to 300 mg/m². Preferably, the paclitaxel drug is administered at a single dose of 50 mg/m² to 200 mg/m².

In one embodiment, the paclitaxel drug is administered once daily, once every two days, once every three days, once every five days, once a week, once every two weeks, once every three weeks, or once every four weeks.

Preferably, the compound is administered at a single dose of 5 mg, 8 mg, 10 mg, 12 mg, or 15 mg, and is administered once daily. Preferably, paclitaxel for injection (albumin bound) is administered at a single dose of 260 mg/m², and is administered once every three weeks. Preferably, paclitaxel for injection (albumin bound) is administered at a single dose of 100 mg/m²-125 mg/m², and is administered once a week.

### Definition

The "halogen" described herein refers to fluorine, chlorine, bromine, iodine, and the like, and preferably fluorine and chlorine.

The "halogenated" described herein means that any hydrogen atom in a substituent can be substituted with one or more identical or different halogen atoms. "Halogen" is defined as above.

The "cyano" described herein refers to -CN group.

The "amino" described herein refers to -NH₂ group.

The "C₁₋₄ alkyl" described herein refers to linear or branched alkyl derived from an alkane moiety having 1 to 4 carbon atoms by removing one hydrogen atom, such as methyl, ethyl, *n*-propyl, isopropyl, *n*-butyl, isobutyl, *sec*-butyl, or *tert*-butyl. The "C₁₋₃ alkyl" refers to the above alkyl having 1 to 3 carbon atoms.

The "C₃₋₆ cycloalkyl" described herein refers to a monocyclic cycloalkyl or bicyclic cycloalkyl system or a polycyclic cycloalkyl system having 3 to 6 carbon atoms. These groups are saturated but not aromatic, including monocyclic and fused structures which can be formed, unless otherwise specified. Examples of the C₃₋₆ cycloalkyl include, but are not limited to: cyclopropyl, cyclobutyl, cyclopentyl, and cyclohexyl.

The "5-11 membered heterocyclyl" described herein refers to a non-aromatic cyclic group having 5 to 11 ring carbon atoms, wherein at least one ring carbon atom is substituted with one or more heteroatoms selected from O, S, and N, preferably 1 to 3 heteroatoms. Moreover, the case where ring-forming atoms including carbon atoms, nitrogen atoms, and sulfur atoms may be oxidized is included.

The "heterocyclyl" refers to a monocyclic heterocyclyl or bicyclic heterocyclyl system or a polycyclic heterocyclyl system, including saturated and partially saturated heterocyclyl, but not including aromatic rings. Unless otherwise specified, the "5-11 membered heterocyclyl" described herein includes monocyclic and fused structures which can be formed.

The monocyclic heterocyclyl may be 5-7 membered heterocyclyl, 5-6 membered heterocyclyl, 5-6 membered oxygen-containing heterocyclyl, 5-6 membered nitrogen-containing heterocyclyl, 5-6 membered saturated heterocyclyl, etc. Examples of the 5-6 membered monocyclic heterocyclyl described herein include, but are not limited to, tetrahydrofuranyl, tetrahydropyrrolyl, tetrahydrothienyl, imidazolidinyl, pyrazolidinyl, 1,2-oxazolidinyl, 1,3-oxazolidinyl, 1,2-thiazolidinyl, 1,3-thiazolidinyl, tetrahydro-2*H*-pyranyl, tetrahydro-2*H*-thiopyranyl, piperidinyl, piperazinyl, morpholinyl, 1,4-dioxanyl, 1,4-oxathianyl, 4,5-dihydroisoxazolyl, 4,5-dihydrooxazolyl, 2,5-dihydrooxazolyl, 2,3-dihydrooxazolyl, 3,4-dihydro-2*H*-pyrrolyl, 2,3-dihydro-1*H*-pyrrolyl, 2,5-dihydro-1*H-*imidazolyl, 4,5-dihydro-1*H*-imidazolyl, 4,5-dihydro-1*H*-pyrazolyl, 4,5-dihydro-3*H-*pyrazolyl, 4,5-dihydrothiazolyl, 2,5-dihydrothiazolyl, 2*H*-pyranyl, 4*H*-pyranyl, 2*H-*thiopyranyl, 4*H*-thiopyranyl, 2,3,4,5-tetrahydropyridyl, 1,2-isoxazolyl, 1,4-isoxazolyl, 6*H-*1,3-oxazinyl, etc.

The fused heterocyclyl includes ortho-fused heterocyclyl, spiro-heterocyclyl, and bridged heterocyclyl, which may be saturated, partially saturated, or unsaturated, but non-aromatic. Unless otherwise specified, the 7-11 membered fused heterocyclyl described herein includes ortho-fused, spiro-, and bridged structures which can be formed.

The ortho-fused heterocyclyl may be 7-11 membered ortho-fused cyclyl, preferably 7-11 membered saturated ortho-fused cyclyl, and examples thereof include, but are not limited to: 3,6-diazabicyclo[3.2.0]heptyl, 3,8-diazabicyclo[4.2.0]octyl, 3,7-diazabicyclo[4.2.0]octyl, octahydropyrrolo[3,4-*c*]pyrrolyl, octahydropyrrolo[3,4-*b*]pyrrolyl, octahydropyrrolo[3,4-*b*][1,4]oxazinyl, octahydro-1*H*-pyrrolo[3,4-*c*]pyridyl, 2,3-dihydrobenzofuran-2-yl, 2,3-dihydrobenzofuran-3-yl, indolin-1-yl, indolin-2-yl, indolin 3-yl, 2,3-dihydrobenzothien-2-yl, octahydro-1*H*-indolyl, and octahydrobenzofuranyl.

The spiro-heterocyclyl may be 7-11 membered spiro-heterocyclyl, preferably 7-11 membered saturated spiro-heterocyclyl, and examples thereof include, but are not limited to:

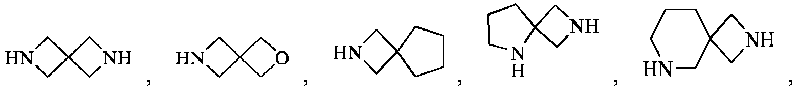

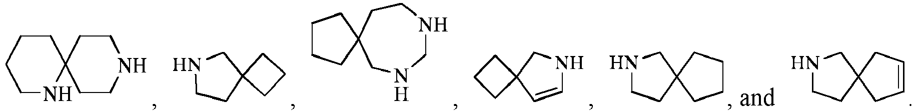

The bridged heterocyclyl may be 7-11 membered bridged heterocyclyl, preferably 7-11 membered saturated bridged heterocyclyl, and examples thereof include, but are not limited to:

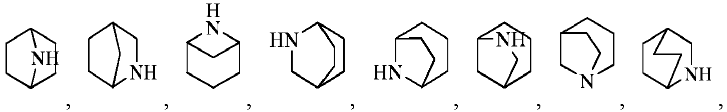

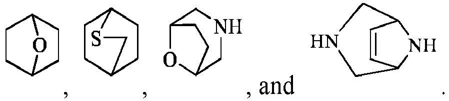

The "pharmaceutically acceptable salt" described herein refers to a pharmaceutically acceptable addition salt of acid and base and a solvate. Such pharmaceutically acceptable salts include salts of the following acids: hydrochloric acid, phosphoric acid, hydrobromic acid, sulfuric acid, sulfurous acid, formic acid, toluenesulfonic acid, methanesulfonic acid, nitric acid, benzoic acid, citric acid, tartaric acid, maleic acid, hydroiodic acid, alkanoic acid (such as acetic acid, HOOC-(CH₂)ₙ-COOH (wherein n = an integer of 0 to 4)), etc. Such pharmaceutically acceptable salts further include salts of the following bases: sodium, potassium, calcium, ammonium, etc. Those skilled in the art know a variety of pharmaceutically acceptable non-toxic addition salts.

All numerical ranges described herein include both endpoints of the ranges, all integers within the ranges, and subranges formed by these integers. For example, "5-11 membered" includes 5, 6, 7, 8, 9, 10, or 11 membered, "5-6 membered" includes 5 or 6 membered, and "7-11 membered" includes 7, 8, 9, 10, 11 membered, etc.

"One or more" as described herein with respect to a substituent refers to the number of substituents with which all positions can be chemically substituted in the substituted group, preferably 1 to 6, more preferably 1 to 5, more preferably 1 to 3, and more preferably 1 to 2.

The "crystal form" described herein may be prepared from a compound of general formula (I) by conventional methods for preparing a crystal form used in the art.

The "stereoisomer" of the compound of general formula (I) described herein means that an enantiomer can be formed when asymmetric carbon atoms are present in the compound of general formula (I); a cis-trans isomer can be formed when a carbon-carbon double bond or a ring structure is present in the compound; a tautomer can be formed when a ketone or oxime is present in the compound. All enantiomers, diastereomers, racemates, cis-trans isomers, tautomers, geometric isomers, and epimers of the compound of general formula (I) as well as mixtures thereof are included in the scope of the present invention.

The preparation of the compound of general formula (I) described herein can be found in the detailed description of WO2018108079A1.

The term "synergistic effect" refers to an effect produced by two active ingredients (for example, a compound of general formula (I) and the paclitaxel drug), whose positive effects include: an effect greater than an additive effect, additional beneficial effects (for example, additional therapeutic effects not previously observed in either ingredient), reduced side effects, a combined therapeutic effect produced by a non-effective dosage of one or both of the first and second active ingredients, and a more synergistic effect of the two active ingredients. The positive effects of the synergistic effect are not limited thereto, and other positive effects may also be included.

The term "has a therapeutic effect in clinic" means that clinical patients have prolonged progression-free survival (PFS), prolonged overall survival (OS), improved objective response rate (ORR), improved disease control rate (DCR), reduced number and/or extent of adverse effects, decreased distant metastasis rates, and decreased local control rates, etc. Specifically, in some specific embodiments of the present application, especially in the specific examples of the present application, the objective response rate of a patient with HER2-negative or triple negative breast cancer in a clinical trial is up to about 15% or more, preferably up to about 20% or more, further preferably up to about 25% or more, more preferably up to about 30% or more, and especially up to 35% or more; the progression-free survival of the patient is up to 3 months or more, preferably up to about 5 months or more, and further preferably up to about 7 months or more; the overall survival is up to about 9 months or more, preferably up to about 15 months or more, further preferably up to about 20 months or more, more preferably up to about 25 months, and especially up to 35 months or more.

The term "breast cancer/HER2-negative breast cancer/triple negative breast cancer after the failure of standard treatment" refers to disease progression during treatment or after the last treatment, or intolerance during treatment due to toxic side effects. "Standard treatment" includes first-line or second-line treatment means such as surgery, radiotherapy, and chemotherapy.

The term "advanced breast cancer/HER2-negative breast cancer/triple negative breast cancer" refers to breast cancer/HER2-negative breast cancer/triple negative breast cancer that is refractory to radical resection due to distant metastasis or severe local infiltration.

The term "metastatic breast cancer/HER2-negative breast cancer/triple negative breast cancer" refers to breast cancer/HER2-negative breast cancer/triple negative breast cancer that spreads from a body site where it originates (a major site) to other body sites.

The term "recurrent breast cancer/HER2-negative breast cancer/triple negative breast cancer" is breast cancer/HER2-negative breast cancer/triple negative breast cancer that regenerates at an initial site or a distant site after response to an initial treatment (for example, surgery).

The present invention provides a group of technical schemes as follows:
1. A pharmaceutical composition, comprising: (a) a compound of general formula (I) or a pharmaceutically acceptable salt, a stereoisomer or a crystal form thereof, and (b) a paclitaxel drug, 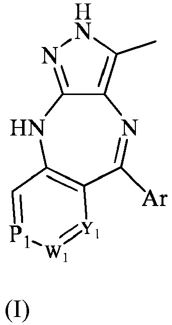
   wherein Ar is phenyl optionally substituted with 1 to 3 R⁶, and each R⁶ is independently selected from hydrogen, amino, cyano, halogen, C₁₋₄ alkyl, and trifluoromethyl;
   Y₁ is CR³;
   P₁ is CR⁴;
   W₁ is N;
   R³ is hydrogen or C₁₋₄ alkyl;
   R⁴ is -(CH₂)ₙ-(5-11) membered heterocyclyl, wherein n = an integer of 0 to 6, a ring-forming S atom (if any) in the heterocyclyl is optionally oxidized to S(O) or S(O)₂, a ring-forming C atom (if any) in the heterocyclyl is optionally oxidized to C(O), and the heterocyclyl is optionally substituted with one or more substituents independently selected from C₁₋₃ alkyl and C₃₋₆ cycloalkyl.
2. The pharmaceutical composition according to technical scheme 1, wherein
   Ar is phenyl optionally substituted with 1 to 3 R⁶, and each R⁶ is independently selected from hydrogen and halogen;
   Y₁ is CR³; R³ is hydrogen;
   P₁ is CR⁴; R⁴ is selected from -(CH₂)ₙ-(5-6) membered monocyclic heterocyclyl and -(CH₂)ₙ-(7-11) membered fused heterocyclyl, wherein n = an integer of 0 to 6, a ring-forming S atom (if any) in the heterocyclyl is optionally oxidized to S(O) or S(O)₂, a ring-forming C atom (if any) in the heterocyclyl is optionally oxidized to C(O), and the heterocyclyl is optionally substituted with one or more substituents independently selected from C₁₋₃ alkyl and C₃₋₆ cycloalkyl;
   W₁ is N.
3. The pharmaceutical composition according to technical scheme 2, wherein R⁴ is 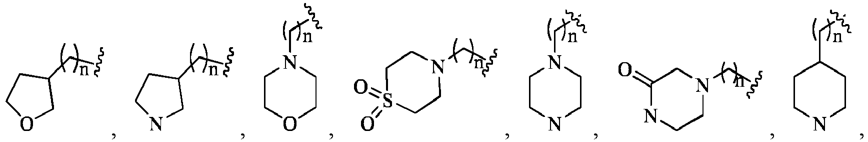 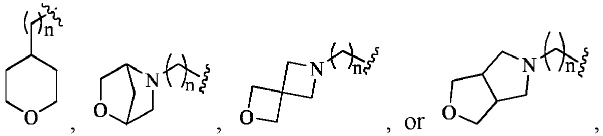 and n = an integer of 0 to 3, wherein the heterocyclyl in R⁴ is optionally substituted with one or more substituents independently selected from C₁₋₃ alkyl and C₃₋₆ cycloalkyl.
4. The pharmaceutical composition according to technical scheme 3, comprising one or more of the following compounds or pharmaceutically acceptable salts, stereoisomers or crystal forms thereof: 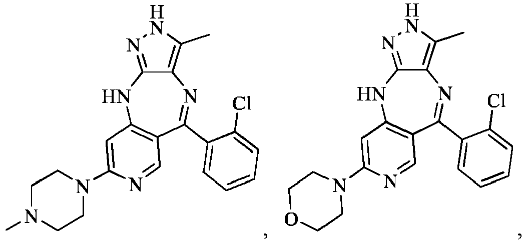 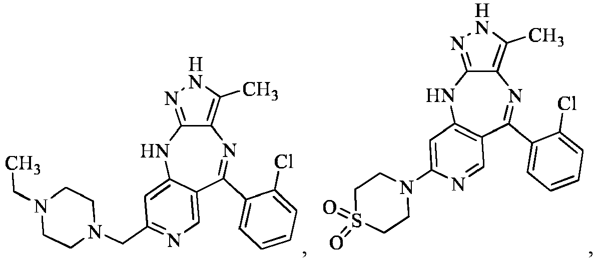 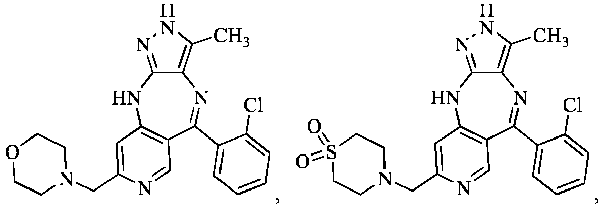 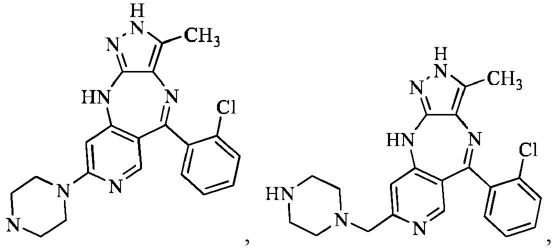 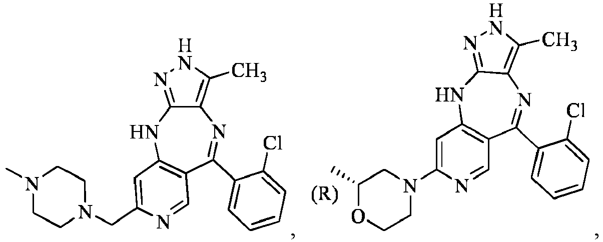 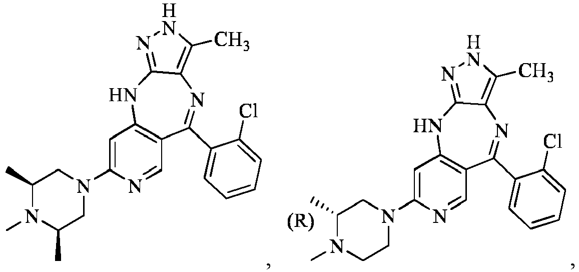 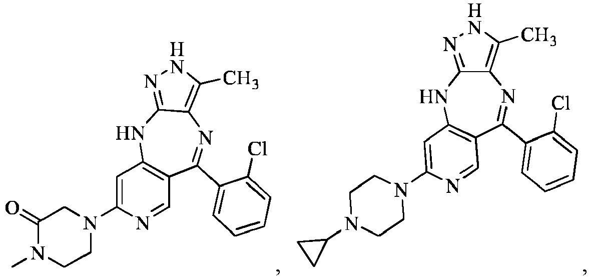 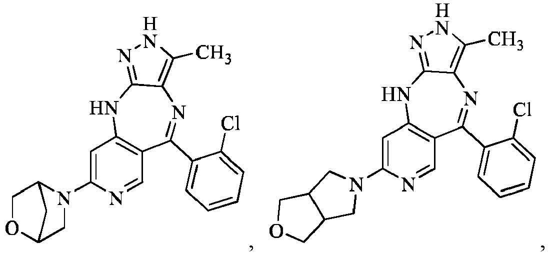 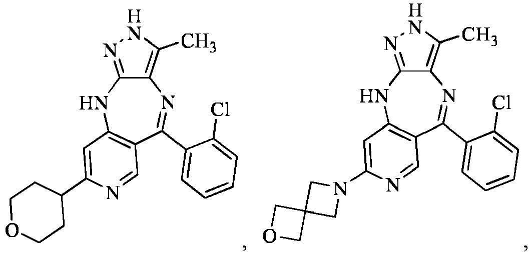 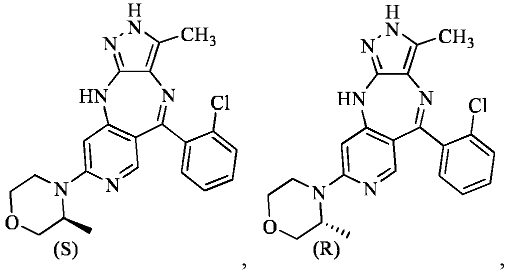 and 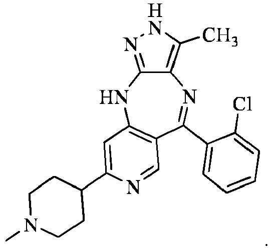
5. The pharmaceutical composition according to any one of technical schemes 1 to 4, wherein the paclitaxel drug is selected from one or more of paclitaxel, paclitaxel for injection (albumin bound), paclitaxel liposome for injection, and docetaxel.
6. The pharmaceutical composition according to any one of technical schemes 1 to 4, comprising compound 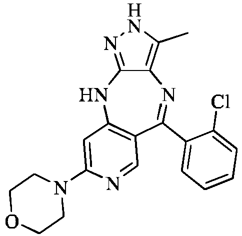 or a pharmaceutically acceptable salt, a stereoisomer or a crystal form thereof; the paclitaxel drug is paclitaxel for injection (albumin bound) or paclitaxel.
7. A combination formulation, comprising the compound or the pharmaceutically acceptable salt, the stereoisomer or the crystal form thereof, and the paclitaxel drug according to any one of technical schemes 1 to 6, and a pharmaceutically acceptable carrier.
8. A kit, comprising the pharmaceutical composition according to any one of technical schemes 1 to 6 or the combination formulation according to technical scheme 7, and a package insert.
9. Use of the pharmaceutical composition according to any one of technical schemes 1 to 6, the combination formulation according to technical scheme 7, or the kit according to technical scheme 8 in the manufacture of a medicament for preventing and/or treating a cancer.
10. The use according to technical scheme 9, wherein the compound of general formula (I) or the pharmaceutically acceptable salt, the stereoisomer or the crystal form thereof, and the paclitaxel drug in the pharmaceutical composition, the combination formulation, or the kit are administered in combination to a cancer patient in therapeutically effective amounts simultaneously, separately, or sequentially.
11. The use according to technical scheme 9 or 10, wherein the cancer is ovarian cancer, endometrial cancer, cervical cancer, head and neck cancer, nasopharyngeal cancer, esophageal cancer, lung cancer, breast cancer, gastric cancer, biliary tract tumor, pancreatic cancer, bladder cancer, or melanoma.
12. The use according to technical scheme 11, wherein the breast cancer is HER2-negative breast cancer.
13. The use according to technical scheme 11, wherein the breast cancer is triple negative breast cancer.

The present invention also provides a group of technical schemes as follows:
1. A pharmaceutical composition, comprising or consisting of: (a) a compound of general formula (I) or a pharmaceutically acceptable salt, a stereoisomer or a crystal form thereof, and (b) a paclitaxel drug, 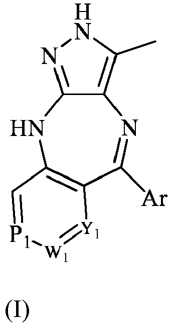
   wherein Ar is phenyl optionally substituted with 1 to 3 R⁶, and each R⁶ is independently selected from hydrogen, amino, cyano, halogen, C₁₋₄ alkyl, and trifluoromethyl;
   Y₁ is CR³;
   P₁ is CR⁴;
   W₁ is N;
   R³ is hydrogen or C₁₋₄ alkyl;
   R⁴ is -(CH₂)ₙ-(5-11) membered heterocyclyl, wherein n = an integer of 0 to 6, a ring-forming S atom (if any) in the heterocyclyl is optionally oxidized to S(O) or S(O)₂, a ring-forming C atom (if any) in the heterocyclyl is optionally oxidized to C(O), and the heterocyclyl is optionally substituted with one or more substituents independently selected from C₁₋₃ alkyl and C₃₋₆ cycloalkyl;
   preferably,
   Ar is phenyl optionally substituted with 1 to 3 R⁶, and each R⁶ is independently selected from hydrogen and halogen;
   Y₁ is CR³, and R³ is hydrogen;
   P₁ is CR⁴, and R⁴ is selected from -(CH₂)ₙ-(5-6) membered monocyclic heterocyclyl and -(CH₂)ₙ-(7-11) membered fused heterocyclyl, wherein n = an integer of 0 to 6, a ring-forming S atom (if any) in the heterocyclyl is optionally oxidized to S(O) or S(O)₂, a ring-forming C atom (if any) in the heterocyclyl is optionally oxidized to C(O), and the heterocyclyl is optionally substituted with one or more substituents independently selected from C₁₋₃ alkyl and C₃₋₆ cycloalkyl;
   W₁ is N.
2. The pharmaceutical composition according to any one of the preceding technical schemes, wherein
   R⁴ is 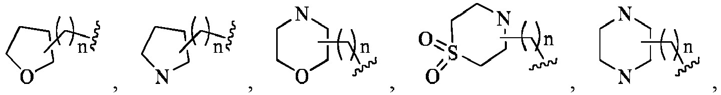 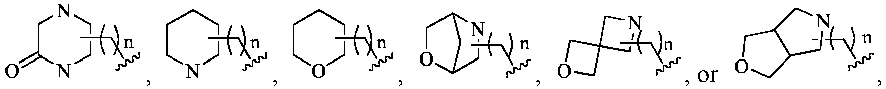 and n = an integer of 0 to 3, wherein the heterocyclyl in R⁴ is optionally substituted with one or more substituents independently selected from C₁₋₃ alkyl and C₃₋₆ cycloalkyl (for example, 1, 2, or 3 substituents independently selected from C₁₋₂ alkyl and C₃ cycloalkyl);
   preferably, R⁴ is 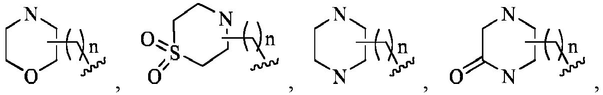 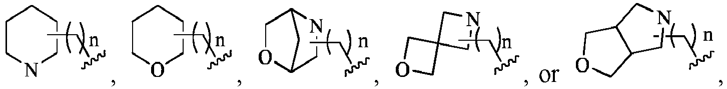 and n = an integer of 0 to 3, wherein the heterocyclyl in R⁴ is optionally substituted with one or more substituents independently selected from C₁₋₃ alkyl and C₃₋₆ cycloalkyl (for example, 1, 2, or 3 substituents independently selected from C₁₋₂ alkyl and C₃ cycloalkyl);
3. The pharmaceutical composition according to any one of the preceding technical schemes, wherein
   R⁴ is 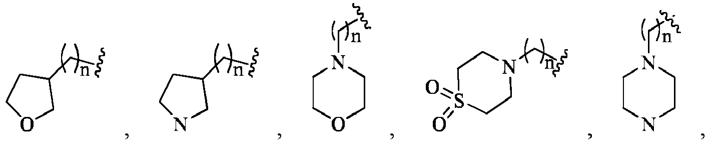 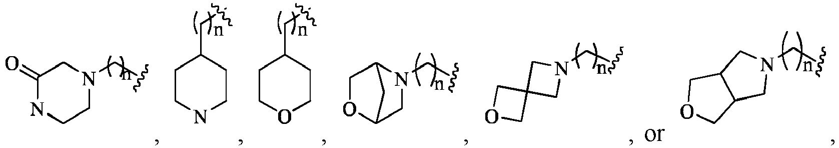 and n = an integer of 0 to 3, wherein the heterocyclyl in R⁴ is optionally substituted with one or more substituents independently selected from C₁₋₃ alkyl and C₃₋₆ cycloalkyl (for example, 1, 2, or 3 substituents independently selected from C₁₋₂ alkyl and C₃ cycloalkyl),
   preferably, R⁴ is 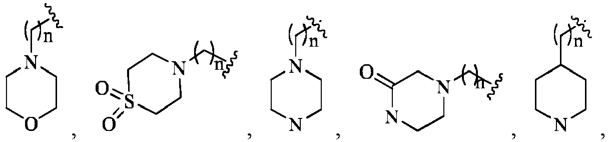 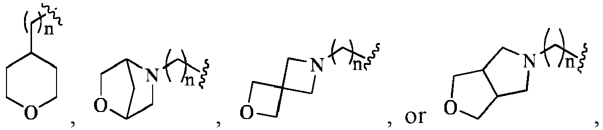 and n = an integer of 0 to 3, wherein the heterocyclyl in R⁴ is optionally substituted with one or more substituents independently selected from C₁₋₃ alkyl and C₃₋₆ cycloalkyl (for example, 1, 2, or 3 substituents independently selected from C₁₋₂ alkyl and C₃ cycloalkyl).
4. A pharmaceutical composition, comprising or consisting of: (a) one or more of the following compounds or pharmaceutically acceptable salts, stereoisomers or crystal forms thereof, and (b) a paclitaxel drug: 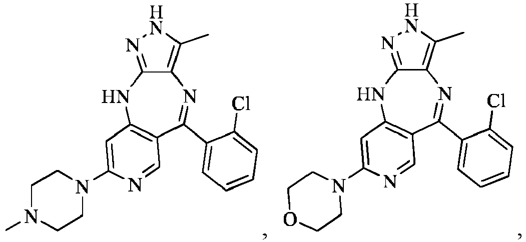 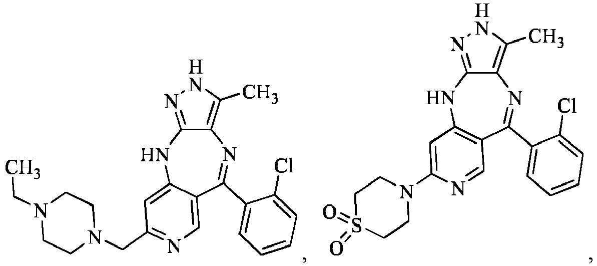 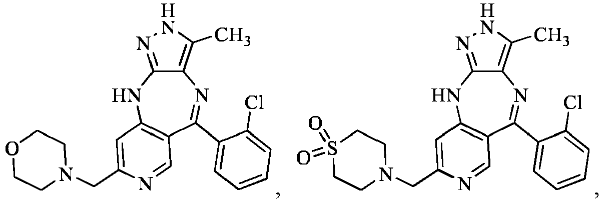 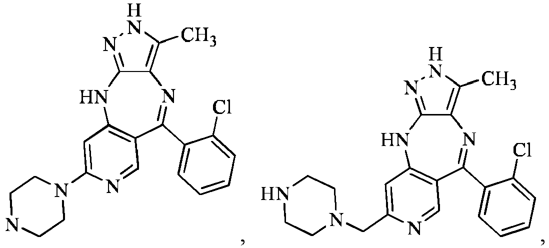 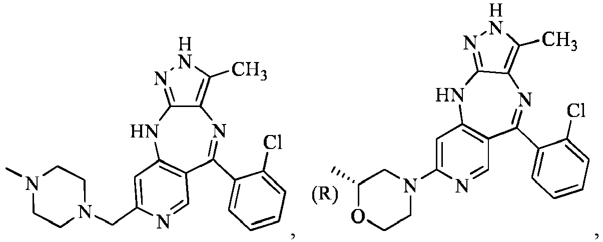 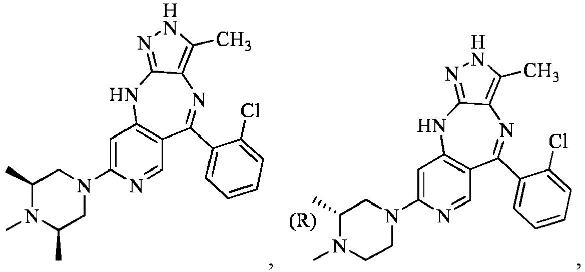 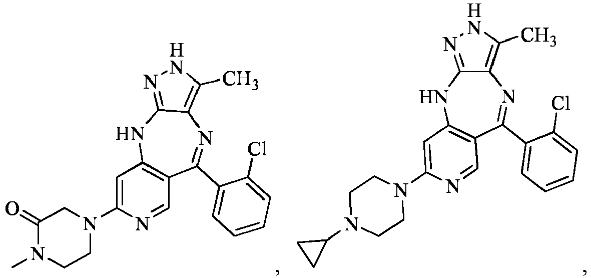 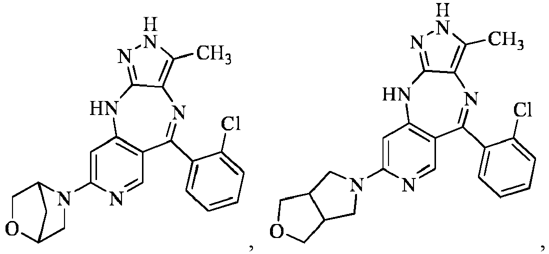 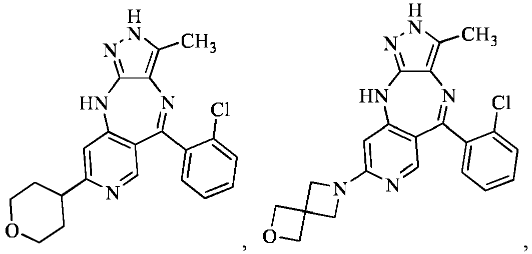 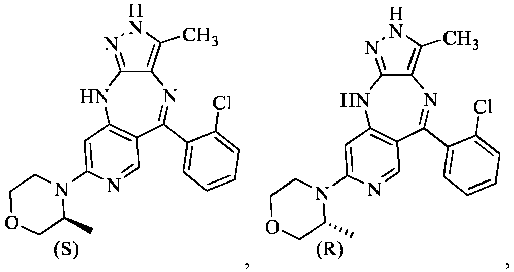 and 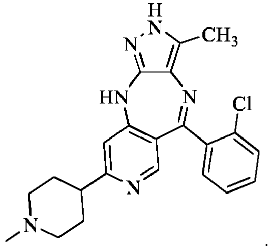
5. The pharmaceutical composition according to any one of the preceding technical schemes, wherein the paclitaxel drug is selected from paclitaxel and analogs and formulations thereof.
6. The pharmaceutical composition according to any one of the preceding technical schemes, wherein the paclitaxel drug is selected from one or more of paclitaxel, docetaxel, and cabazitaxel.
7. The pharmaceutical composition according to any one of the preceding technical schemes, wherein the paclitaxel drug is selected from one or more of paclitaxel, paclitaxel for injection (albumin bound), paclitaxel liposome for injection, and docetaxel.
8. The pharmaceutical composition according to any one of the preceding technical schemes, wherein component (a) is 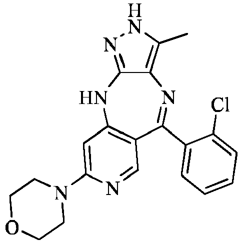 or a pharmaceutically acceptable salt, a stereoisomer or a crystal form thereof; the paclitaxel drug is paclitaxel for injection (albumin bound) or paclitaxel (preferably, the paclitaxel drug is paclitaxel for injection (albumin bound), such as Abraxane; more preferably, component (a) is administered orally at a dose of 8 mg, 10 mg, or 12 mg once daily, with 28 consecutive days of administration being one cycle, and component (b) is administered by intravenous infusion at a dose of 50 mg/m²-400 mg/m² (for example, 100 mg/m²) for approximately 30 min-120 min on days 1, 8, and 15 of each cycle, with 28 days being one cycle).
9. A pharmaceutical composition, comprising or consisting of: (a) one or more of the following compounds or pharmaceutically acceptable salts, stereoisomers or crystal forms thereof, and (b) a paclitaxel drug (preferably, the paclitaxel drug being selected from one or more of paclitaxel, paclitaxel for injection (albumin bound), paclitaxel liposome for injection, and docetaxel): 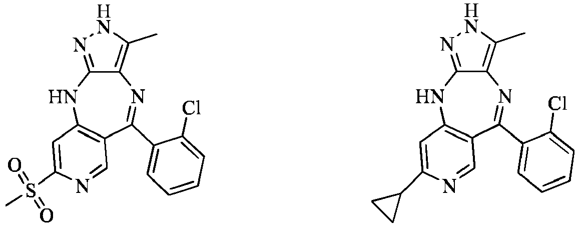 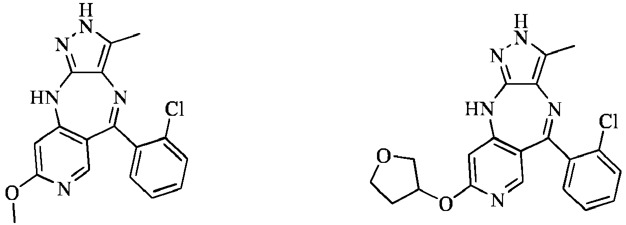 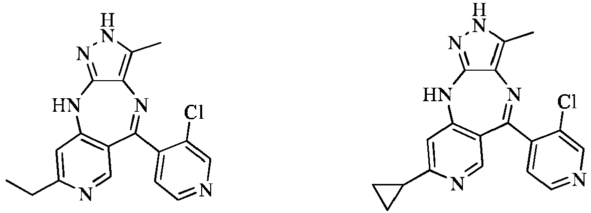 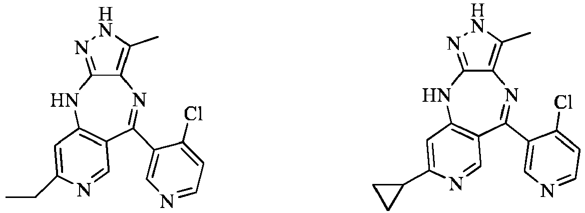 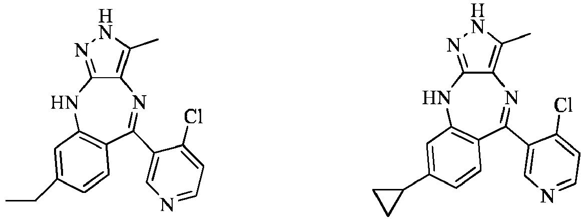 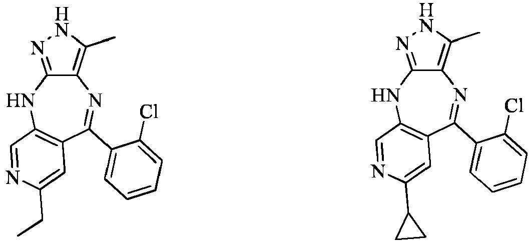 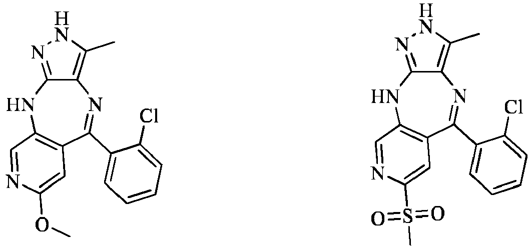 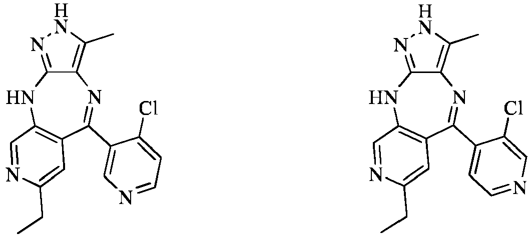 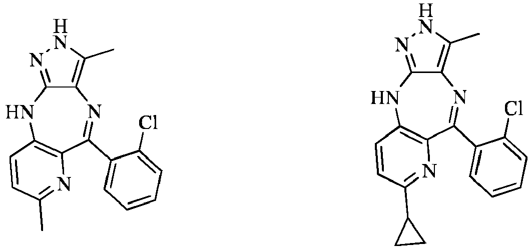 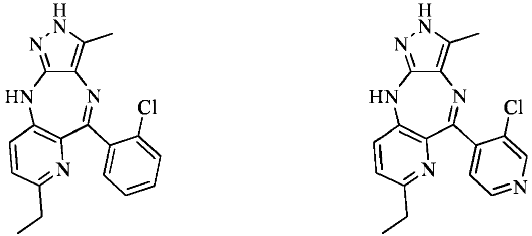 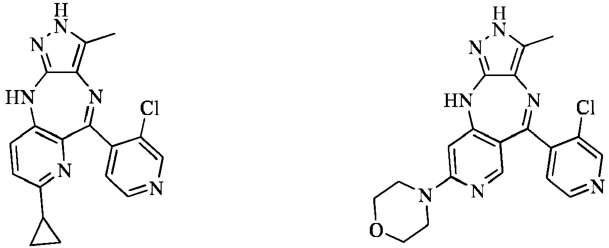 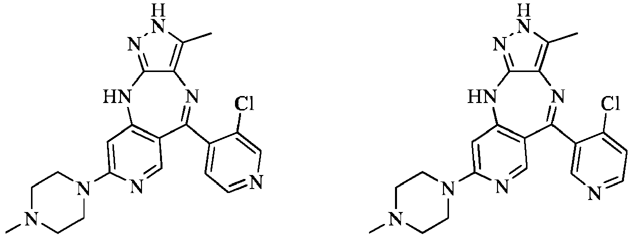 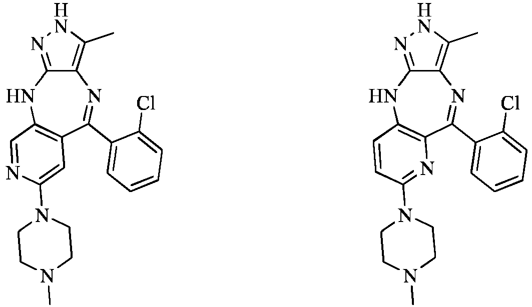 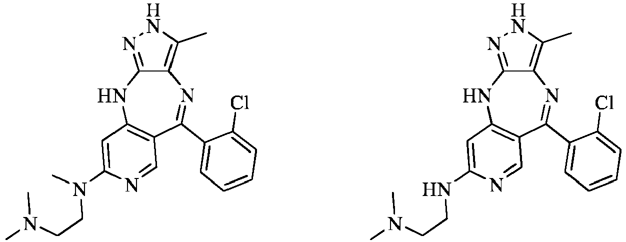 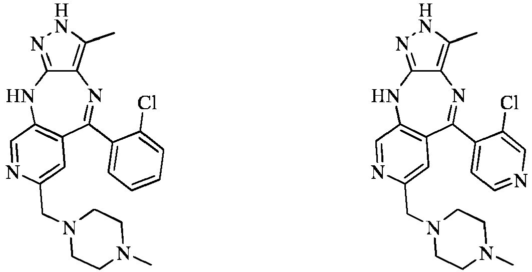
10. The pharmaceutical composition according to any one of the preceding technical schemes, wherein the paclitaxel drug is selected from: Paclitaxel Kabi, Taxol, Aosu, Anzatax, Tesu, Funeng, Zisu, Zexin, Anxiao, Furuitai, Lipusu, Keruifei, Qiluruibei, Abraxane, Aiyue, Keaili, Taxotere, Aomingrun, Aisu, Duopafei, Chenhuan, Yiyouruikang, Xicun, Siqudi, cabazitaxel, polyglutamic acid paclitaxel, BMS-275183, ortataxel, BMS-184476, larotaxel, paclitaxel trevatide, Salutaxel, Conmutaxol, felotaxel, BMS-188797, milataxel, tesetaxel, and docosahexaenoic acid-paclitaxel.
11. The pharmaceutical composition according to any one of the preceding technical schemes, wherein component (a) and component (b) are in a weight ratio of 1:10 to 10:1, for example, 15:25.
12. A combination formulation, comprising the pharmaceutical composition according to any one of technical schemes 1 to 11 and a pharmaceutically acceptable carrier, wherein preferably, the pharmaceutical composition and the carrier are in a weight ratio of 1:1000 to 1000:1.
13. A kit, comprising the pharmaceutical composition according to any one of technical schemes 1 to 11 or the combination formulation according to technical scheme 12, and a package insert.
14. Use of the pharmaceutical composition according to any one of technical schemes 1 to 11, the combination formulation according to claim 12, or the kit according to claim 13 in the manufacture of a medicament for preventing and/or treating a cancer.
15. The use according to technical scheme 14, wherein component (a) and component (b) in the pharmaceutical composition, the combination formulation, or the kit are administered in combination to a cancer patient in therapeutically effective amounts simultaneously, separately, or sequentially.
16. The use according to technical scheme 14 or 15, wherein the cancer is ovarian cancer, endometrial cancer, cervical cancer, head and neck cancer, nasopharyngeal cancer, esophageal cancer, lung cancer, breast cancer, gastric cancer, biliary tract tumor, pancreatic cancer, bladder cancer, or melanoma; preferably, the breast cancer is HER2-negative breast cancer or triple negative breast cancer, more preferably, the breast cancer is one after the failure of standard treatment, and/or is metastatic, and/or recurrent, or locally advanced, or advanced breast cancer or HER2-negative breast cancer or triple negative breast cancer.
17. The pharmaceutical composition according to any one of technical schemes 1 to 11, the combination formulation according to technical scheme 12, or the kit according to technical scheme 13 for use in the prevention and/or treatment of a cancer.
18. The pharmaceutical composition, the combination formulation, or the kit according to technical scheme 17, wherein component (a) and component (b) in the pharmaceutical composition, the combination formulation, or the kit are administered in combination to a cancer patient in therapeutically effective amounts simultaneously, separately, or sequentially.
19. The pharmaceutical composition, the combination formulation, or the kit according to technical scheme 17 or 18, wherein the cancer is ovarian cancer, endometrial cancer, cervical cancer, head and neck cancer, nasopharyngeal cancer, esophageal cancer, lung cancer, breast cancer, gastric cancer, biliary tract tumor, pancreatic cancer, bladder cancer, or melanoma; preferably, the breast cancer is HER2-negative breast cancer or triple negative breast cancer, more preferably, the breast cancer is one after the failure of standard treatment, and/or is metastatic, and/or recurrent, or locally advanced, or advanced breast cancer or HER2-negative breast cancer or triple negative breast cancer.
20. A method for preventing and/or treating a cancer, including administering to a cancer patient in need thereof a therapeutically effective amount of the pharmaceutical composition according to any one of technical schemes 1 to 11, the combination formulation according to technical scheme 12, or the kit according to technical scheme 13.
21. The method according to technical scheme 20, wherein component (a) and component (b) in the pharmaceutical composition, the combination formulation, or the kit are administered in combination to a cancer patient in therapeutically effective amounts simultaneously, separately, or sequentially.
22. The method according to technical scheme 20 or 21, wherein the cancer is ovarian cancer, endometrial cancer, cervical cancer, head and neck cancer, nasopharyngeal cancer, esophageal cancer, lung cancer, breast cancer, gastric cancer, biliary tract tumor, pancreatic cancer, bladder cancer, or melanoma; preferably, the breast cancer is HER2-negative breast cancer or triple negative breast cancer, more preferably, the breast cancer is one after the failure of standard treatment, and/or is metastatic, and/or recurrent, or locally advanced, or advanced breast cancer or HER2-negative breast cancer or triple negative breast cancer.
23. The use according to any one of technical schemes 14 to 16, or the pharmaceutical composition, the combination formulation, or the kit according to any one of technical schemes 17 to 19, or the method according to any one of technical schemes 20 to 22, wherein
   component (b) is administered by injection, for example, intravenously, for more than 30 min; component (a) is administered orally, further orally on an empty stomach; or
   component (a) is administered at a single dose of 3 mg to 20 mg, for example, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, or 20 mg, and is administered once daily or once every two days, preferably once daily;
   component (b) is administered at a single dose of 50 mg/m² to 400 mg/m², for example, 50 mg/m², 55 mg/m², 60 mg/m², 65 mg/m², 70 mg/m², 75 mg/m², 80 mg/m², 85 mg/m², 90 mg/m², 95 mg/m², 100 mg/m², 105 mg/m², 110 mg/m², 115 mg/m², 120 mg/m², 125 mg/m², 130 mg/m², 135 mg/m², 140 mg/m², 145 mg/m², 150 mg/m², 155 mg/m², 160 mg/m², 165 mg/m², 170 mg/m², 175 mg/m², 180 mg/m², 185 mg/m², 190 mg/m², 195 mg/m², 200 mg/m², 205 mg/m², 210 mg/m², 215 mg/m², 220 mg/m², 225 mg/m², 230 mg/m², 235 mg/m², 240 mg/m², 245 mg/m², 250 mg/m², 255 mg/m², 260 mg/m², 265 mg/m², 270 mg/m², 275 mg/m², 280 mg/m², 285 mg/m², 290 mg/m², 295 mg/m², 300 mg/m², 305 mg/m², 310 mg/m², 315 mg/m², 320 mg/m², 325 mg/m², 330 mg/m², 335 mg/m², 340 mg/m², 345 mg/m², 350 mg/m², 355 mg/m², 360 mg/m², 365 mg/m², 370 mg/m², 375 mg/m², 380 mg/m², 385 mg/m², 390 mg/m², 395 mg/m², or 400 mg/m², or any value between 50 mg/m² and 400 mg/m²; preferably, component (b) is administered at a single dose of 100 mg/m² to 300 mg/m², more preferably 50 mg/m² to 200 mg/m²;
   component (b) is administered once daily, once every two days, once every three days, once every five days, once a week, once every two weeks, once every three weeks, or once every four weeks;
   component (a) is administered at a single dose of 5 mg, 8 mg, 10 mg, 12 mg, or 15 mg, and is administered once daily; or
   component (b) is paclitaxel for injection (albumin bound), which is administered at a single dose of 260 mg/m², and is administered once every three weeks; preferably, component (b) is paclitaxel for injection (albumin bound), which is administered at a single dose of 100 mg/m²-125 mg/m², and is administered once a week.

### BENEFICIAL EFFECTS OF PRESENT INVENTION

The compound of general formula (I) or the pharmaceutically acceptable salt, the stereoisomer or the crystal form thereof of the present invention has a synergistic effect when being administered in combination with a paclitaxel drug for treating a cancer, and particularly has a significant effect on ovarian cancer, endometrial cancer, cervical cancer, head and neck cancer, nasopharyngeal cancer, esophageal cancer, lung cancer, breast cancer, gastric cancer, biliary tract tumor, pancreatic cancer, bladder cancer, or melanoma. Furthermore, compared with the clinical standard treatment, paclitaxel monotherapy, the combined administration of the compound of general formula (I) or the pharmaceutically acceptable salt, the stereoisomer or the crystal form thereof of the present invention with a paclitaxel drug has significantly improved efficacy in the treatment of triple negative breast cancer, and has better clinical application potential.

In order to make the objective, technical schemes, and advantages of the present invention more apparent, the present invention is further described in detail below. It should be apparent that the examples described herein are only some examples of the present invention, but not all examples. Based on the examples of the present invention, all other examples obtained by those of ordinary skill in the art without creative work shall fall within the protection scope of the present invention.

The abbreviations and English expressions used in the present invention have the following meanings:

**Table 2: Abbreviations and meanings**

| Abbreviation/English | Meaning |
|---|---|
| DMSO | Dimethyl sulfoxide |
| MC | Methylcellulose |
| Qd | Once-daily administration |
| Qw | Once-weekly administration |

### Experimental Example 1: In vivo pharmacodynamic study of the compound of the present invention combined with paclitaxel on triple negative breast cancer patient-derived xenograft (PDX) subcutaneous tumor model

Test samples: compound 29 of the present invention, which has a structure shown above, and the preparation method can be found in the detailed description of WO2018108079A1.

Animals, information on the patient from whom the tumor mass was derived, and other materials:
a human triple negative breast cancer tumor sample BC408, derived from a female patient;
female nude mice, 6- to 8-week-old, derived from Charles River Lab;
paclitaxel injection: Paclitaxel Kabi^{®} 6 mg/mL, derived from Frenesius Kabi.

### Test method:

### 1. Construction and grouping of tumor-bearing mice

The tumor masses were inoculated into the right back of the mice, the animals were weighed before administration, and tumor volumes were measured. Grouping was designed in blocks according to the tumor volume, with 8 mice per group except for the vehicle control, which contained 6 mice.

### 2. Administration regimen

Administration was carried out as in Table 3 below.

**Table 3: Administration regimen**

| Group | Dosage (mg/kg) | Route of administration | Frequency of administration | Administration period |
|---|---|---|---|---|
| Vehicle control | 0 | Oral intragastric administration | Qd * 5 days/week | 5 weeks |
| Compound 29 | 15 | Oral intragastric administration | Qd * 5 days/week | 5 weeks |
| Paclitaxel injection | 25 | Intraperitoneal injection | Qw | 5 weeks |
| Compound 29 + paclitaxel injection | 15 +25 | Oral intragastric administration Intraperitoneal injection | Qd * 5 days/week Qw | 5 weeks |

| | | | | |
|---|---|---|---|---|
| Notes: vehicle control: 5% DMSO + 0.5% MC; formula of compound 29: 5% DMSO + 0.5% MC; formula of paclitaxel injection: normal saline. | | | | |

### Experimental observation index

Animals were monitored daily for health status and mortality, body weight and tumor volume were measured twice a week, and samples were collected after the last administration. The therapeutic effect of tumor volume was evaluated by TGI%, wherein the relative tumor inhibition rate TGI (%): TGI = 1-T/C (%). T/C % is the relative tumor proliferation rate, i.e., the percentage of the relative tumor volume in the treatment and control groups at a given time point. T and C are relative tumor volumes (RTVs) of the treatment and control groups at a given time point, respectively. The calculation formula is as follows: T/C % = TRTV / CRTV × 100% (TRTV: mean RTV for treatment group; CRTV: mean RTV for vehicle control group; RTV = Vt / V0, wherein V0 is the tumor volume of the animal at the time of grouping, and Vt is the tumor volume of the animal after treatment).

The results are shown in Table 4 below:

**Table 4: Effect of compound 29 of the present invention combined with paclitaxel on tumor growth of a human triple negative breast cancer BC408 subcutaneous xenograft tumor model**

| Group | Number of animals | Tumor volume (mm³) | TGI (%) |
|---|---|---|---|
| Vehicle control | 6 | 2056 | -- |
| Compound 29 | 8 | 666 | 72 |
| Paclitaxel | 8 | 1132 | 58 |
| Compound 29 + paclitaxel | 8 | 111 | 94 |

As can be seen from the experimental results in Table 4, compound 29 had a significant inhibitory effect on the triple negative breast cancer patient-derived xenograft (PDX) subcutaneous tumor model, which was significantly superior to paclitaxel used in clinical standard treatment, and the efficacy was significantly improved by the combination of compound 29 with paclitaxel, indicating that the combined administration of the compound of the present invention and paclitaxel is suitable for clinical treatment of triple negative breast cancer, and has good clinical application potential.

### Experimental Example 2: Clinical study of the compound of the present invention combined with paclitaxel for injection (albumin bound) in the treatment of patients with breast cancer

Objective: the preliminary efficacy of treatment of patients with HER2-negative breast cancer receiving compound 29 of the present invention combined with paclitaxel for injection (albumin bound) was assessed.

Inclusion criteria: patients with HER2-negative breast cancer (including patients with triple negative breast cancer) that had failed and metastasized after standard treatment.

Test drug: compound 29, prepared as described in WO2018108079A1, with a specification of 4 mg, 5 mg, and 6 mg.

Test drug: paclitaxel for injection (albumin bound) was Abraxane^{®}.

### Administration regimen:

compound 29 was administered orally at a prescribed dose (such as 8 mg, 10 mg, or 12 mg) once daily, with 28 consecutive days of administration being one cycle;
paclitaxel for injection (albumin bound) was administered by intravenous infusion at a prescribed dose (such as 100 mg/m²) for approximately 30 min on days 1, 8, and 15 of each cycle, with 28 days being one cycle.

Clinical assessment: the assessment was made by preliminary efficacy parameters such as objective response rate (ORR), disease control rate (DCR), progression-free survival (PFS), and overall survival (OS).

The research shows that the combination of compound 29 with paclitaxel for injection (albumin bound) has a therapeutic effect in clinic.

The above description is only for the purpose of illustrating preferred examples of the present invention, and is not intended to limit the scope of the present invention. Any modifications, equivalents, improvements, and the like made without departing from the spirit and principle of the present invention should be included in the protection scope of the present invention.

## Claims

1. A pharmaceutical composition, comprising: (a) a compound of general formula (I) or a pharmaceutically acceptable salt, a stereoisomer or a crystal form thereof, and (b) a paclitaxel drug, 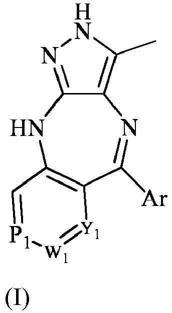
wherein Ar is phenyl optionally substituted with 1 to 3 R⁶, and each R⁶ is independently selected from hydrogen, amino, cyano, halogen, C₁₋₄ alkyl, and trifluoromethyl;
Y₁ is CR³;
P₁ is CR⁴;
W₁ is N;
R³ is hydrogen or C₁₋₄ alkyl;
R⁴ is -(CH₂)ₙ-(5-11) membered heterocyclyl, wherein n = an integer of 0 to 6, a ring-forming S atom (if any) in the heterocyclyl is optionally oxidized to S(O) or S(O)₂, a ring-forming C atom (if any) in the heterocyclyl is optionally oxidized to C(O), and the heterocyclyl is optionally substituted with one or more substituents independently selected from C₁₋₃ alkyl and C₃₋₆ cycloalkyl;
preferably,
Ar is phenyl optionally substituted with 1 to 3 R⁶, and each R⁶ is independently selected from hydrogen and halogen;
Y₁ is CR³, and R³ is hydrogen;
P₁ is CR⁴, and R⁴ is selected from -(CH₂)ₙ-(5-6) membered monocyclic heterocyclyl and -(CH₂)ₙ-(7-11) membered fused heterocyclyl, wherein n = an integer of 0 to 6, a ring-forming S atom (if any) in the heterocyclyl is optionally oxidized to S(O) or S(O)₂, a ring-forming C atom (if any) in the heterocyclyl is optionally oxidized to C(O), and the heterocyclyl is optionally substituted with one or more substituents independently selected from C₁₋₃ alkyl and C₃₋₆ cycloalkyl;
W₁ is N.

2. The pharmaceutical composition according to the preceding claim 1, wherein
R⁴ is 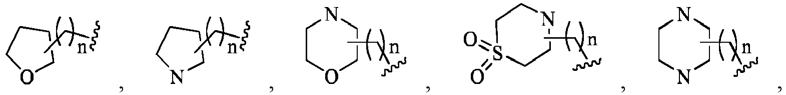 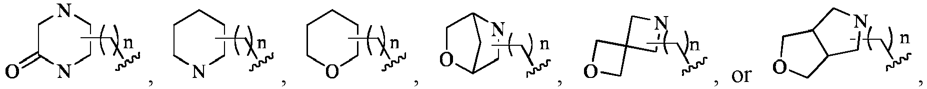 and n = an integer of 0 to 3, wherein the heterocyclyl in R⁴ is optionally substituted with one or more substituents independently selected from C₁₋₃ alkyl and C₃₋₆ cycloalkyl (for example, 1, 2, or 3 substituents independently selected from C₁₋₂ alkyl and C₃ cycloalkyl);
preferably, R⁴ is 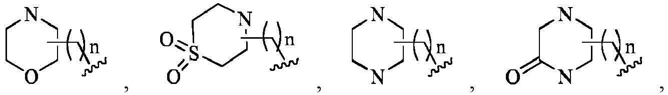 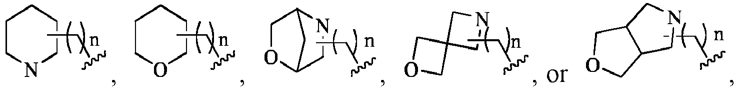 and n = an integer of 0 to 3, wherein the heterocyclyl in R⁴ is optionally substituted with one or more substituents independently selected from C₁₋₃ alkyl and C₃₋₆ cycloalkyl (for example, 1, 2, or 3 substituents independently selected from C₁₋₂ alkyl and C₃ cycloalkyl);
particularly preferably, R⁴ is 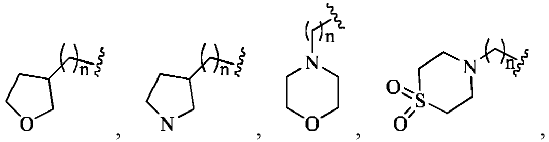 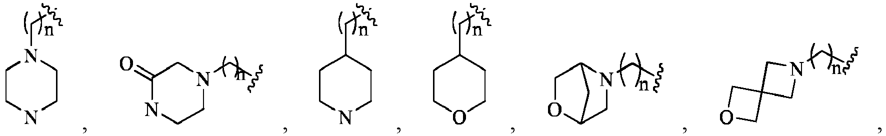 or 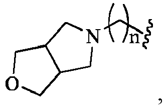 and n = an integer of 0 to 3, wherein the heterocyclyl in R⁴ is optionally substituted with one or more substituents independently selected from C₁₋₃ alkyl and C₃₋₆ cycloalkyl (for example, 1, 2, or 3 substituents independently selected from C₁₋₂ alkyl and C₃ cycloalkyl);
more particularly preferably, R⁴ is 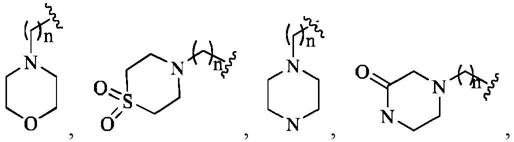 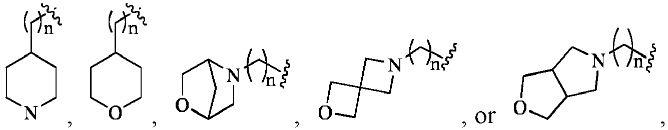 and n = an integer of 0 to 3, wherein the heterocyclyl in R⁴ is optionally substituted with one or more substituents independently selected from C₁₋₃ alkyl and C₃₋₆ cycloalkyl (for example, 1, 2, or 3 substituents independently selected from C₁₋₂ alkyl and C₃ cycloalkyl).

3. A pharmaceutical composition, comprising: (a) one or more of the following compounds or pharmaceutically acceptable salts, stereoisomers or crystal forms thereof, and (b) a paclitaxel drug: 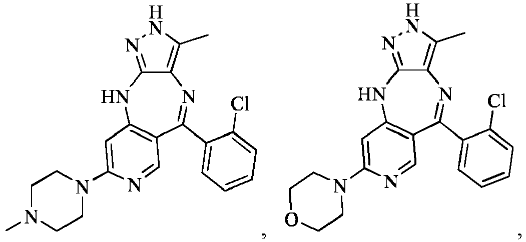 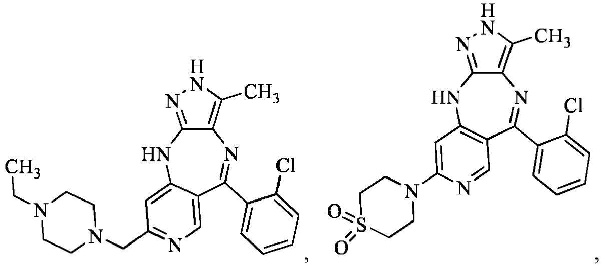 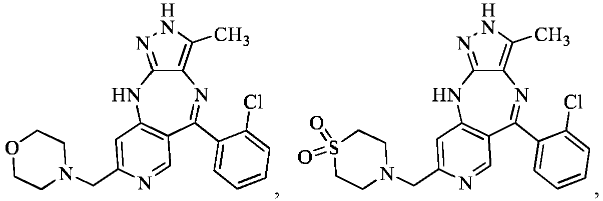 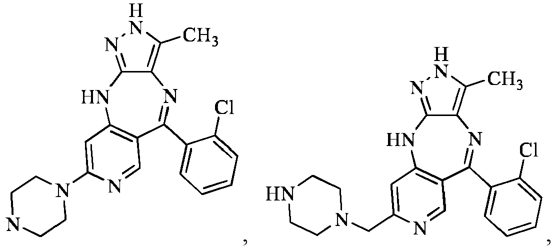 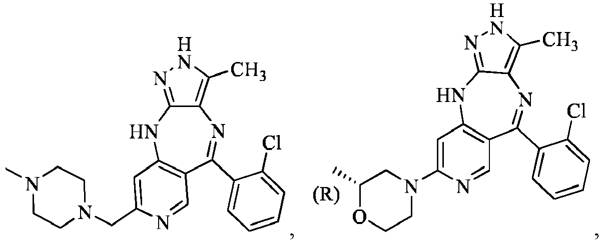 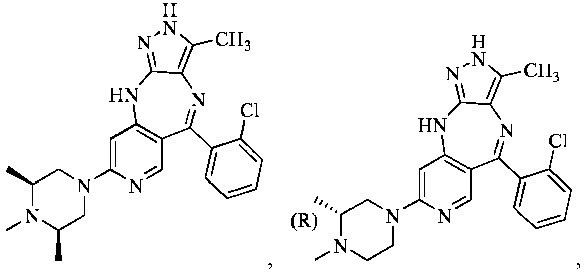 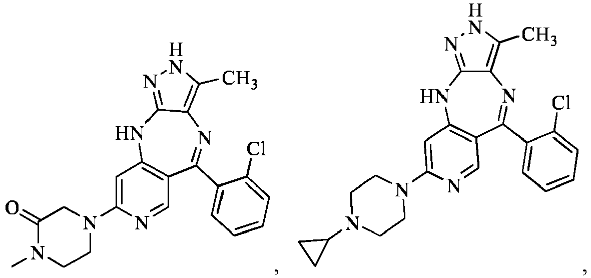 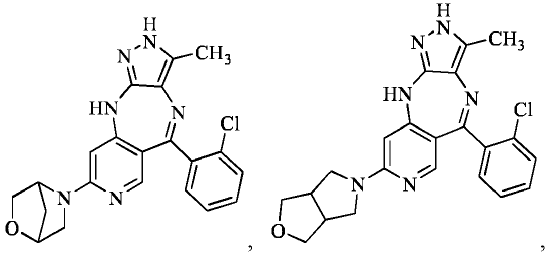 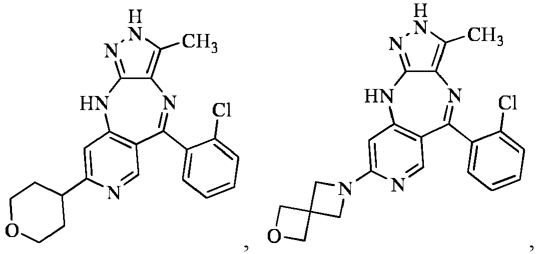 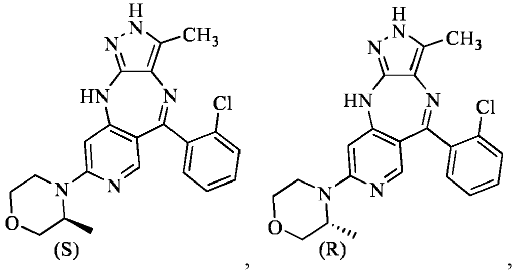 and

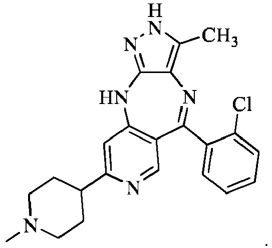

4. The pharmaceutical composition according to any one of the preceding claims 1 to 3, wherein the paclitaxel drug is selected from paclitaxel and analogs and formulations thereof.

5. The pharmaceutical composition according to any one of the preceding claims 1 to 3, wherein the paclitaxel drug is selected from one or more of paclitaxel, docetaxel, and cabazitaxel.

6. The pharmaceutical composition according to any one of the preceding claims 1 to 3, wherein the paclitaxel drug is selected from one or more of paclitaxel, paclitaxel for injection (albumin bound), paclitaxel liposome for injection, and docetaxel.

7. The pharmaceutical composition according to any one of the preceding claims 1 to 3, wherein component (a) is 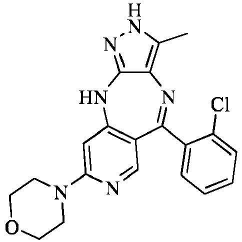 or a pharmaceutically acceptable salt, a stereoisomer or a crystal form thereof; the paclitaxel drug is paclitaxel for injection (albumin bound) or paclitaxel.

8. The pharmaceutical composition according to any one of the preceding claims 1 to 3, wherein component (a) and component (b) are in a weight ratio of 1: 10 to 10: 1, for example, 15:25.

9. The pharmaceutical composition according to any one of the preceding claims 1 to 3, wherein the paclitaxel drug is selected from: Paclitaxel Kabi, Taxol, Aosu, Anzatax, Tesu, Funeng, Zisu, Zexin, Anxiao, Furuitai, Lipusu, Keruifei, Qiluruibei, Abraxane, Aiyue, Keaili, Taxotere, Aomingrun, Aisu, Duopafei, Chenhuan, Yiyouruikang, Xicun, Siqudi, cabazitaxel, polyglutamic acid paclitaxel, BMS-275183, ortataxel, BMS-184476, larotaxel, paclitaxel trevatide, Salutaxel, Conmutaxol, felotaxel, BMS-188797, milataxel, tesetaxel, and docosahexaenoic acid-paclitaxel.

10. The pharmaceutical composition according to any one of the preceding claims 1 to 3, wherein the paclitaxel drug is selected from: Paclitaxel Kabi, Taxol, Aosu, Anzatax, Tesu, Funeng, Zisu, Zexin, Anxiao, Furuitai, Lipusu, Keruifei, Qiluruibei, Abraxane, Aiyue, Keaili, Taxotere, Aomingrun, Aisu, Duopafei, Chenhuan, Yiyouruikang, Xicun, Siqudi, cabazitaxel, polyglutamic acid paclitaxel, BMS-275183, ortataxel, BMS-184476, larotaxel, paclitaxel trevatide, Salutaxel, Conmutaxol, felotaxel, BMS-188797, milataxel, tesetaxel, and docosahexaenoic acid-paclitaxel;
component (a) and component (b) are in a weight ratio of 1: 10 to 10: 1, for example, 15:25.

11. A combination formulation, comprising the pharmaceutical composition according to any one of claims 1 to 10 and a pharmaceutically acceptable carrier, wherein preferably, the pharmaceutical composition and the carrier are in a weight ratio of 1: 1000 to 1000:1.

12. A kit, comprising the pharmaceutical composition according to any one of claims 1 to 10 or the combination formulation according to claim 11, and a package insert.

13. Use of the pharmaceutical composition according to any one of claims 1 to 10, the combination formulation according to claim 11, or the kit according to claim 12 in the manufacture of a medicament for preventing and/or treating a cancer.

14. The use according to claim 13, wherein component (a) and component (b) in the pharmaceutical composition, the combination formulation, or the kit are administered in combination to a cancer patient in therapeutically effective amounts simultaneously, separately, or sequentially.

15. The use according to claim 13 or 14, wherein the cancer is ovarian cancer, endometrial cancer, cervical cancer, head and neck cancer, nasopharyngeal cancer, esophageal cancer, lung cancer, breast cancer, gastric cancer, biliary tract tumor, pancreatic cancer, bladder cancer, or melanoma; preferably, the breast cancer is HER2-negative breast cancer or triple negative breast cancer, more preferably, the breast cancer is one after the failure of standard treatment, and/or is metastatic, and/or recurrent, or locally advanced, or advanced breast cancer or HER2-negative breast cancer or triple negative breast cancer.

16. The use according to any one of claims 13 to 15, wherein
component (b) is administered by injection, for example, intravenously, for more than 30 min; component (a) is administered orally, further orally on an empty stomach; or
component (a) is administered at a single dose of 3 mg to 20 mg, for example, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, or 20 mg, and is administered once daily or once every two days, preferably once daily;
component (b) is administered at a single dose of 50 mg/m² to 400 mg/m², for example, 50 mg/m², 55 mg/m², 60 mg/m², 65 mg/m², 70 mg/m², 75 mg/m², 80 mg/m², 85 mg/m², 90 mg/m², 95 mg/m², 100 mg/m², 105 mg/m², 110 mg/m², 115 mg/m², 120 mg/m², 125 mg/m², 130 mg/m², 135 mg/m², 140 mg/m², 145 mg/m², 150 mg/m², 155 mg/m², 160 mg/m², 165 mg/m², 170 mg/m², 175 mg/m², 180 mg/m², 185 mg/m², 190 mg/m², 195 mg/m², 200 mg/m², 205 mg/m², 210 mg/m², 215 mg/m², 220 mg/m², 225 mg/m², 230 mg/m², 235 mg/m², 240 mg/m², 245 mg/m², 250 mg/m², 255 mg/m², 260 mg/m², 265 mg/m², 270 mg/m², 275 mg/m², 280 mg/m², 285 mg/m², 290 mg/m², 295 mg/m², 300 mg/m², 305 mg/m², 310 mg/m², 315 mg/m², 320 mg/m², 325 mg/m², 330 mg/m², 335 mg/m², 340 mg/m², 345 mg/m², 350 mg/m², 355 mg/m², 360 mg/m², 365 mg/m², 370 mg/m², 375 mg/m², 380 mg/m², 385 mg/m², 390 mg/m², 395 mg/m², or 400 mg/m², or any value between 50 mg/m² and 400 mg/m²; preferably, component (b) is administered at a single dose of 100 mg/m² to 300 mg/m², more preferably 50 mg/m² to 200 mg/m²;
component (b) is administered once daily, once every two days, once every three days, once every five days, once a week, once every two weeks, once every three weeks, or once every four weeks;
component (a) is administered at a single dose of 5 mg, 8 mg, 10 mg, 12 mg, or 15 mg, and is administered once daily; or
component (b) is paclitaxel for injection (albumin bound), which is administered at a single dose of 260 mg/m², and is administered once every three weeks; preferably, component (b) is paclitaxel for injection (albumin bound), which is administered at a single dose of 100 mg/m²-125 mg/m², and is administered once a week.
